# EUROPEAN PATENT SPECIFICATION

(11) **EP 4 051 702 B1**
(45) Date of publication and mention of the grant of the patent: **08.04.2026**
(21) Application number: 20883299.8
(22) Date of filing: 30.10.2020
(51) Int. Cl.: C07K 14/705, C12Q 1/6883, G01N 33/68, A61B 5/02, A61B 5/055

(54) **THERANOSTICS FOR HYPERTENSION INDUCED MYOCARDIAL MICROBLEEDS**
THERANOSTIKA FÜR BLUTHOCHDRUCK-INDUZIERTE MYOKARDIALE MIKROBLUTUNGEN
THÉRANOSTIQUE POUR MICROSAIGNEMENTS MYOCARDIQUES INDUITS PAR L'HYPERTENSION

(30) Priority: 31.10.2019 US 201962928717 P
(43) Date of publication of application: 07.09.2022
(73) Proprietor: Cedars-Sinai Medical Center, Los Angeles, CA 90048 (US); Board of Supervisors of Louisiana State University and Agricultural and Mechanical College, Baton Rouge, LA 70808 (US)
(72) Inventor: DHARMAKUMAR, Rohan, Moorpark, California 93021 (US); FRANCIS, Joseph, Baton Rouge, Louisiana 70820 (US)
(74) Representative: Hutter, Anton
(86) International application number: PCT/US2020/058335
(87) International publication number: WO 2021/087344

(56) References cited:
- US-A1- 2011 044 524
- US-A1- 2012 263 362
- US-A1- 2016 183 815
- US-B1- 8 886 283
- HE ET AL.: "Deferoxamine inhibits microglial activation, attenuates blood-brain barrier disruption, rescues dendritic damage, and improves spatial memory in a mouse model of microhemorrhages", JOURNAL OF NEUROCHEMISTRY, vol. 138, no. 3, 1 August 2016 (2016-08-01), GB , pages 436 - 447, XP055932660, ISSN: 0022-3042, DOI: 10.1111/jnc.13657
- KALI ET AL.: "Chronic Manifestation of Postreperfusion Intramyocardial Hemorrhage as Regional Iron Deposition : A Cardiovascular Magnetic Resonance Study With Ex Vivo Validation", CIRCULATION. CARDIOVASCULAR IMAGING, vol. 6, no. 2, 1 March 2013 (2013-03-01), US , pages 218 - 228, XP055932666, ISSN: 1941-9651, DOI: 10.1161/CIRCIMAGING.112.000133

## Description

### CROSS REFERENCE TO RELATED APPLICATIONS

This application includes a claim of priority under 35 U.S.C. §119(e) to U.S. provisional patent application no. 62/928,717, filed October 31, 2019, q.v..

### FIELD OF THE INVENTION

The invention relates to methods of cardiovascular imaging for detecting, diagnosing and/or prognosing various symptoms associated with hypertension and cardiovascular diseases and to the treatment thereof.

### BACKGROUND

The following description includes information that may be useful in understanding the present invention.

Many people suffer from hypertension or are at risk of developing hypertension. Hypertension can lead to a various adverse outcomes including cardiovascular disease, cardiac arrhythmia, sudden cardiac death, and death. As such there is a need for methods for detecting, diagnosing, and/or treating symptoms associated with hypertension to reduce adverse outcomes in people with hypertension, especially for people with hypertension who are also at risk of developing or suffering from cardiovascular diseases or heart-related illnesses.

US 2016/183815 A1 discloses assessment of iron deposition post myocardial infarction as a marker of myocardial hemorrhage.

### SUMMARY OF THE INVENTION

The following embodiments and aspects thereof are described and illustrated in conjunction with compositions and methods which are meant to be exemplary and illustrative, not limiting in scope.

In various embodiments, methods are provided for identifying and/or assessing at least one microbleed in a heart of a subject, comprising: obtaining at least one image of the heart of the subject; and detecting at least one iron deposit in the heart of the subject from the at least one image, wherein detection of the iron deposit in the heart of the subject is indicative of the at least one microbleed in the heart of the subject. In other embodiments, methods are provided for identifying and/or assessing at least one microbleed in a heart of a subject, comprising: detecting and/or measuring the presence of at least one iron deposit in the heart of the subject by measuring from blood of the subject a level of a hemoglobin breakdown product or a level of a ferroptosis marker, wherein detection of the iron deposit in the heart of the subject is indicative of the at least one microbleed in the heart of the subject.

In various embodiments, the at least one microbleed in the heart is induced by or associated with hypertension. In some embodiments, the subject has hypertension. In some embodiments, the subject has been diagnosed with hypertension. In some embodiments, the subject has secondary hypertension caused by another condition, therefore the subject also has or is diagnosed with chronic kidney disease, polycystic kidney disease, coarctation of aorta in the heart, adrenal disorder, hyperthyroidism, narrowing artery to the kidney, sleep disorders, or pheochromocytoma. In some embodiments, the subject has taken or takes blood pressure medications. In some embodiments, the subject has stress-induced myocardiopathy. In some embodiments, the subject does not have myocardial infarction or reperfusion therapy-induced hemorrhage; in some further aspects, the subject does not have hemorrhagic myocardial infarction; in some further aspects, the subject has not undergone reperfusion therapy, and therefore would not have reperfusion hemorrhage. In some embodiments, the subject has not had a myocardial infarction or a reperfusion therapy in the past 3 days, 7 days, 1 month, 2 months, 3 months, 4 months, 5 months, 6 months, 7 months, 8 months, 9 months, 10 months, 11 months, 12 months, 2 years, 3 years, or 5 years. In some embodiments, a subject in need of one or more methods disclosed herein is a subject with any one or more characteristics above; and in some embodiments, a subject in need thereof desires to identify, detect, prognose or diagnose myocardial microbleeds.

In various embodiments, methods are provided for detecting at least one microbleed in a heart of a subject, comprising: detecting and/or measuring the presence of at least one iron deposit in the heart of the subject by performing an imaging of the heart and/or measuring from blood of the subject a level of a hemoglobin breakdown product or a level of a ferroptosis marker, wherein detection of the at least one iron deposit in the heart of the subject is indicative of and/or is a detection of the at least one microbleed in the heart of the subject.

In various embodiments, methods are provided for prognosing at least one microbleed in a heart of a subject, comprising: detecting and/or measuring the presence of at least one iron deposit in the heart of the subject by performing an imaging of the heart and/or measuring from blood of the subject a level of a hemoglobin breakdown product or a level of a ferroptosis marker, wherein detection of the at least one iron deposit in the heart of the subject is a prognosis of the at least one microbleed in the heart of the subject.

In various embodiments, methods are provided for diagnosing at least one microbleed in a heart of a subject, comprising: detecting and/or measuring the presence of at least one iron deposit in the heart of the subject by performing an imaging of the heart and/or measuring from blood of the subject a level of a hemoglobin breakdown product or a level of a ferroptosis marker, wherein detection of the at least one iron deposit in the heart of the subject is a diagnosis of the at least one microbleed in the heart of the subject.

In various embodiments, methods are provided for assessing and/or determining the risk of developing at least one microbleed in a heart of a subject, comprising: detecting and/or measuring the presence of at least one iron deposit in the heart of the subject by performing an imaging of the heart and/or measuring from blood of the subject a level of a hemoglobin breakdown product or a level of a ferroptosis marker, wherein detection of the at least one iron deposit in the heart of the subject is indicative of an increased risk of developing at least one microbleed in the heart of the subject.

In various embodiments, methods are provided for identifying a subject with a cardiac microbleed, wherein the subject has hypertension, the methods comprising selecting a subject diagnosed with hypertension or suffering from hypertension, and performing a clinical imaging scan of at least a portion of the heart of the subject to detect iron-containing deposits in the portion of the heart. Exemplary clinical imaging scans include single-photon emission computed tomography (SPECT), positron emission tomography (PET), computer tomography (CT), ultrasound (US), magnetic resonance imaging (MRI), and a combination thereof. One or more of these imaging techniques can tag the breakdown products of hemoglobin or proteins associated with ferroptosis.

In various embodiments, methods are provided for identifying and/or assessing at least one microbleed in at least one portion of a heart of a subject, comprising: detecting at least one iron deposit in the at least one portion of the heart of the subject by performing a clinical imaging of the least one portion of the heart, wherein detection of the at least one iron deposit in the at least one portion of the heart of the subject is indicative of the at least one microbleed in the at least one portion of the heart of the subject.

In various embodiments, methods are provided for detecting at least one microbleed in at least one portion of a heart of a subject, comprising: detecting at least one iron deposit in the at least one portion of the heart of the subject by performing a clinical imaging of the least one portion of the heart, wherein detection of the at least one iron deposit in the at least one portion of the heart of the subject is indicative of and/or is a detection of the at least one microbleed in the at least one portion of the heart of the subject.

In various embodiments, methods are provided for prognosing at least one microbleed in at least one portion of a heart of a subject, comprising: detecting at least one iron deposit in the at least one portion of the heart of the subject by performing a clinical imaging of the least one portion of the heart, wherein detection of the at least one iron deposit in the at least one portion of the heart of the subject is a prognosis of the at least one microbleed in the at least one portion of the heart of the subject.

In various embodiments, methods are provided for diagnosing at least one microbleed in at least one portion of a heart of a subject, comprising: detecting at least one iron deposit in the at least one portion of the heart of the subject by performing a clinical imaging of the least one portion of the heart, wherein detection of the at least one iron deposit in the at least one portion of the heart of the subject is a diagnosis of the at least one microbleed in the at least one portion of the heart of the subject.

In various embodiments, methods are provided for assessing and/or determining the risk of developing at least one microbleed in at least one portion of a heart of a subject, comprising: detecting at least one iron deposit in the at least one portion of the heart of the subject by performing a clinical imaging of the least one portion of the heart, wherein detection of the at least one iron deposit in the at least one portion of the heart of the subject is indicative of an increased risk of the subject developing at least one microbleed in the at least one portion of the heart of the subject.

In various embodiments, methods are provided for identifying and/or assessing at least one microbleed in at least one heart tissue of a subject, comprising: detecting at least one iron deposit in the at least one heart tissue of the subject by performing a clinical imaging of the least one heart tissue, wherein detection of the at least one iron deposit in the at least one heart tissue is indicative of the at least one microbleed in the at least one heart tissue of the subject.

In various embodiments, methods are provided for detecting at least one microbleed in at least one heart tissue of a subject, comprising: detecting at least one iron deposit in the at least one heart tissue of the subject by performing a clinical imaging of the least one heart tissue, wherein detection of the at least one iron deposit in the at least one heart tissue is indicative of and/or is a detection of the at least one microbleed in the at least one heart tissue of the subject.

In various embodiments, methods are provided for prognosing at least one microbleed in at least one heart tissue of a subject, comprising: detecting at least one iron deposit in the at least one heart tissue of the subject by performing a clinical imaging of the least one heart tissue, wherein detection of the at least one iron deposit in the heart is a prognosis of the at least one microbleed in the heart of the subject.

In various embodiments, methods are provided for diagnosing at least one microbleed in at least one heart tissue of a subject, comprising: detecting at least one iron deposit in the at least one heart tissue of the subject by performing a clinical imaging of the least one heart tissue, wherein detection of the at least one iron deposit in the at least one heart tissue is a diagnosis of the at least one microbleed in the at least one heart tissue of the subject.

In various embodiments, methods are provided for assessing and/or determining the risk of developing at least one microbleed in at least one heart tissue of a subject, comprising: detecting at least one iron deposit in the at least one heart tissue of the subject by performing a clinical imaging of the least one heart tissue, wherein detection of the at least one iron deposit in the at least one heart tissue is indicative of an increased risk of the subject developing at least one microbleed in the at least one heart tissue of the subject.

In various embodiments, methods are provided for identifying and/or assessing at least one microbleed in a heart of a subject, comprising: detecting at least one iron deposit in the heart of the subject by performing a clinical imaging of the heart, and comparing the image of the heart of the subject to at least one image from a reference sample, wherein the presence of at least one iron deposit in the heart of the subject relative to the reference sample is indicative of at least one microbleed in the heart of the subject.

In various embodiments, methods are provided for detecting at least one microbleed in a heart of a subject, comprising: detecting at least one iron deposit in the heart of the subject by performing a clinical imaging of the heart, and comparing the image of the heart of the subject to at least one image from a reference sample, wherein the presence of at least one iron deposit in the heart of the subject relative to the reference sample is indicative of at least one microbleed in the heart of the subject.

In various embodiments, methods are provided for prognosing at least one microbleed in a heart of a subject, comprising: detecting at least one iron deposit in the heart of the subject by performing a clinical imaging of the heart, and comparing the image of the heart of the subject to at least one image from a reference sample, wherein the presence of at least one iron deposit in the heart of the subject relative to the reference sample is a prognosis of at least one microbleed in the heart of the subject.

In various embodiments, methods are provided for diagnosing at least one microbleed in a heart of a subject, comprising: detecting at least one iron deposit in the heart of the subject by performing a clinical imaging of the heart, and comparing the image of the heart of the subject to at least one image from a reference sample, wherein the presence of at least one iron deposit in the heart of the subject relative to the reference sample is a diagnosis of at least one microbleed in the heart of the subject.

In various embodiments, methods are provided for assessing and/or determining the risk of developing at least one microbleed in a heart of a subject, comprising: detecting at least one iron deposit in the heart of the subject by performing a clinical imaging of the heart, and comparing the image of the heart of the subject to at least one image from a reference sample, wherein the presence of at least one iron deposit in the heart of the subject relative to the reference sample is indicative of an increased risk of the subject developing at least one microbleed in the heart of the subject.

In various embodiments, methods are provided for determining progression of at least one microbleed in a heart of a subject, comprising: detecting at least one iron deposit in the heart of the subject by performing a clinical imaging of the heart, and comparing the image of the heart of the subject to at least one image from a reference sample, wherein a change in the image of the heart of the subject relative to the at least one image from the reference sample is indicative of progression of at least one microbleed in the heart of the subject.

In various embodiments, methods are provided for identifying and/or assessing cardiovascular disease in a subject, comprising: detecting at least one iron deposit in the heart of the subject by performing a clinical imaging of the heart, wherein detection of the iron deposit in the heart of the subject is indicative of a presence at least one microbleed in the heart of the subject; comparing the image of heart of the subject to at least one image from a reference sample, wherein a change in the image of heart of the subject relative to the at least one image from the reference sample is indicative of cardiovascular disease in the subject.

In various embodiments, methods are provided for detecting cardiovascular disease in a subject, comprising: detecting at least one iron deposit in the heart of the subject by performing a clinical imaging of the heart, wherein detection of the at least one iron deposit in the heart of the subject is indicative of the presence at least one microbleed in the heart of the subject; and comparing the at least one image of the heart of the subject to at least one image from a reference sample, wherein a change in the at least one image of heart of the subject relative to the at least one image from the reference sample is indicative of cardiovascular disease in the subject.

In various embodiments, methods are provided for detecting cardiovascular disease in a subject, comprising: detecting at least one iron deposit in the heart of the subject by performing a clinical imaging of the heart, wherein detection of the at least one iron deposit in the heart of the subject is indicative of the presence at least one microbleed in the heart of the subject; comparing the at least one image of heart of the subject to at least one image from a reference sample, wherein a change in the at least one image of heart of the subject relative to the at least one image from the reference sample is indicative of cardiovascular disease in the subject.

In various embodiments, methods are provided for prognosing cardiovascular disease in a subject, comprising: detecting at least one iron deposit in the heart of the subject by performing a clinical imaging of the heart, wherein detection of the iron deposit in the subject's heart is indicative of the presence at least one microbleed in the subject's heart; and comparing the image of subject's heart to at least one image from a reference sample, wherein a change in the image of subject's heart relative to the at least one image from the reference sample is a prognosis of cardiovascular disease in the subject.

In various embodiments, methods are provided for diagnosing cardiovascular disease in a subject, comprising: detecting at least one iron deposit in the heart of the subject by performing a clinical imaging of the heart, wherein detection of the iron deposit in the subject's heart is indicative of the presence at least one microbleed in the subject's heart; and comparing the image of subject's heart to at least one image from a reference sample, wherein a change in the image of subject's heart relative to the at least one image from the reference sample is a diagnosis of cardiovascular disease in the subject.

In various embodiments, methods are provided for assessing and/or determining the risk of developing cardiovascular disease in a subject, comprising: detecting at least one iron deposit in the heart of the subject by performing a clinical imaging of the heart, wherein detection of the iron deposit in the subject's heart is indicative of the presence at least one microbleed in the subject's heart; and comparing the image of subject's heart to at least one image from a reference sample, wherein a change in the image of subject's heart relative to the at least one image from the reference sample is indicative of an increased risk of the subject developing cardiovascular disease in the subject.

In various embodiments, methods are provided for determining progression of cardiovascular disease in a subject, comprising: detecting at least one iron deposit in the heart of the subject by performing a clinical imaging of the heart, wherein detection of the iron deposit in the subject's heart is indicative of the presence at least one microbleed in the subject's heart; and comparing the image of subject's heart to at least one image from a reference sample, wherein a change in the image of subject's heart relative to the at least one image from the reference sample is indicative of progression of cardiovascular disease in the subject.

In various embodiments, the cardiovascular disease can be one, two, or multiple cardiac conditions.

In various embodiments, methods are provided for identifying at least one biomarker of cardiovascular disease in a subject, comprising: detecting at least one iron deposit in the heart of the subject by performing a clinical imaging of the heart; correlating the at least one iron deposit in the at least one image of the heart of the subject to at least one microbleed in the heart of the subject so as to obtain a biomarker signature for the subject; and comparing the biomarker signature from the subject to a biomarker signature from a reference sample, wherein the at least one biomarker of cardiovascular disease is identified.

In various embodiments, methods are provided for identifying and/or assessing cardiovascular disease in a subject, comprising: detecting at least one iron deposit in the heart of the subject by performing a clinical imaging of the heart; correlating the at least one iron deposit in the heart of the subject to at least one microbleed in the heart of the subject so as to obtain a biomarker signature for the subject; and comparing the biomarker signature from the subject to a biomarker signature from a reference sample, wherein a change in the biomarker signature from the subject relative to the biomarker signature from the reference sample is indicative of cardiovascular disease in the subject.

In various embodiments, methods are provided for detecting cardiovascular disease in a subject, comprising: detecting at least one iron deposit in the heart of the subject by performing a clinical imaging of the heart; correlating the at least one iron deposit in the heart of the subject to at least one microbleed in the heart of the subject so as to obtain a biomarker signature for the subject; and comparing the biomarker signature from the subject to a biomarker signature from a reference sample, wherein a change in the biomarker signature from the subject relative to the biomarker signature from the reference sample is indicative of and/or a detection of cardiovascular disease in the subject.

In various embodiments, methods are provided for prognosing cardiovascular disease in a subject, comprising: detecting at least one iron deposit in the heart of the subject by performing a clinical imaging of the heart; correlating the at least one iron deposit in the heart of the subject to at least one microbleed in the heart of the subject so as to obtain a biomarker signature for the subject; and comparing the biomarker signature from the subject to a biomarker signature from a reference sample, wherein a change in the biomarker signature from the subject relative to the biomarker signature from the reference sample is a prognosis of cardiovascular disease in the subject.

In various embodiments, methods are provided for diagnosing cardiovascular disease in a subject, comprising: detecting at least one iron deposit in the heart of the subject by performing a clinical imaging of the heart; correlating the at least one iron deposit in the heart of the subject to at least one microbleed in the heart of the subject so as to obtain a biomarker signature for the subject; and comparing the biomarker signature from the subject to a biomarker signature from a reference sample, wherein a change in the biomarker signature from the subject relative to the biomarker signature from the reference sample is a diagnosis of cardiovascular disease in the subject.

In various embodiments, methods are provided for determining progression cardiovascular disease in a subject, comprising: detecting at least one iron deposit in the heart of the subject by performing a clinical imaging of the heart; correlating the at least one iron deposit in the heart of the subject to at least one microbleed in the heart of the subject so as to obtain a biomarker signature for the subject; and comparing the biomarker signature from the subject to a biomarker signature from a reference sample, wherein a change in the biomarker signature from the subject relative to the biomarker signature from the reference sample is indicative of progression of cardiovascular disease in the subject.

In some embodiments, methods are provided for assessing and/or determining the risk of developing cardiovascular disease in a subject, comprising: detecting at least one iron deposit in the heart of the subject by performing a clinical imaging of the heart; correlating the at least one iron deposit in the heart of the subject to at least one microbleed in the heart of the subject so as to obtain a biomarker signature for the subject; and comparing the biomarker signature from the subject to a biomarker signature from a reference sample, wherein a change in the biomarker signature from the subject relative to the biomarker signature from the reference sample is indicative of an increased risk of the subject developing cardiovascular disease.

In various embodiments, methods are provided for assessing and/or determining the risk of at least one cardiac arrhythmia in a subject, comprising: detecting at least one iron deposit in the heart of the subject by performing a clinical imaging of the heart, wherein the presence of the at least one iron deposit in the heart of the subject is indicative of the at least one microbleed in the heart of the subject, wherein at least one microbleed in the heart of the subject is indicative of an increased risk of at least one cardiac arrhythmia in the subject.

In various embodiments, methods are provided for assessing and/or determining the risk of sudden cardiac death in a subject, comprising: detecting at least one iron deposit in the heart of the subject by performing a clinical imaging of the heart, wherein the presence of the at least one iron deposit in the heart of the subject is indicative of the at least one microbleed in the heart of the subject, wherein at least one microbleed in the heart of the subject is indicative of an increased risk of sudden cardiac death in the subject.

In various embodiments, methods are provided for assessing and/or determining the risk of cardiovascular disease in a subject, comprising: detecting at least one iron deposit in the heart of the subject by performing a clinical imaging of the heart, wherein the presence of the at least one iron deposit in the heart of the subject is indicative of the at least one microbleed in the heart of the subject, wherein at least one microbleed in the heart of the subject is indicative of an increased risk of cardiovascular disease in a subject.

In some embodiments, the subject is human. In some embodiments, the subject has at least one selected from the group consisting of hypertension, hypertensive crisis, acute hypertensive crisis, prolonged hypertension, and a combination thereof. In some embodiments, the subject has myocardiopathy, such as stress-induced myocardiopathy.

Various embodiments provide methods for treating or reducing the severity or risk of a cardiovascular disease associated with myocardial microbleeding in a subject who has hypertension or hypertensive crisis, the methods comprising: administering to the subject an effective amount of a composition comprising an iron chelating agent, a heme-binding protein, a heme-degrading protein, a heme-blocking protein, or a factor effective to increase an amount of a heme-binding protein or a heme-degrading protein.

Further embodiments of these treatment methods include selecting a subject with iron deposits in at least one portion of the heart, and administering to the subject the effective amount of the composition. In some aspects, a subject with iron deposits in the heart is selected and/or identified by performing a clinical imaging scan and/or measuring blood-based markers to detect presence of iron in the heart.

In some embodiments, the method further comprises treating the subject with a treatment and/or selecting a treatment for the subject and/or providing a treatment to the subject and/or administering a treatment to the subject. In some embodiments, the treatment is at least one chelating agent. In some embodiments, the chelating agent is selected from the group consisting of Deferoxamine, Deferasirox, Deferiprone, hinokitiol, dexrazoxane, 2,2'-bipyridyl, and combinations thereof. In some embodiments, the chelating agent is an iron chelator. In some embodiments, the iron chelator is selected from the group consisting of an intracellular iron chelator, extracellular iron chelator, or combination thereof. In some embodiments, the intracellular iron chelator chelates ferrous iron, ferric iron, and combinations thereof. In some embodiments, the extracellular iron chelator chelates ferrous iron, ferric iron, and combinations thereof. In some embodiments, the treatment is selected from the group consisting of at least one heme binding protein, at least one heme degrading protein, at least one heme-blocking agent/protein, and combinations thereof. In some embodiments, the method further comprises administering to the subject an amount of a factor effective to increase an amount of at least one selected from the group consisting of at least one heme binding protein, at least one heme degrading protein, and combinations thereof in the subject. In some embodiments, the heme binding protein is selected from the group consisting of hemopexin, haptoglobin, albumin, ferritin, alpha1-microglobulin, alpha1-antitrypsin, glutathione-S-transferases, heme binding protein / Liver fatty acid binding protein, heme-binding protein 23 / peroxiredoxin, p22 heme binding protein and glyceraldehyde-3-phosphate dehydrogenase, and combinations thereof. In some embodiments, the heme degrading protein is selected from the group consisting of heme oxygenase 1, nuclear factor E2 related factor 2 (Nrf2), and combinations thereof. In some embodiments, the heme-blocking agent/protein is hepcidin. In some embodiments, the factor is selected from FLVCR1a (Feline leukemia virus subgroup C receptor 1a), ABCG2 (ATP-binding cassette subfamily G member 2), FLVCR2, and combinations thereof. In some embodiments, the heme binding protein is a heme scavenger.

In some embodiments, the iron deposit, also referred to as iron-containing deposit, comprises iron oxide. In some embodiments, the iron deposit comprises at least one compound comprising iron. In some embodiments, the compound is selected from the group consisting of heme, heme derivative, and combinations thereof. In some embodiments, the iron is in any oxidation state possible. In some embodiments, the oxidation state is selected from +2, +2/+3, +3, +4, and combinations thereof. In some embodiments, the iron is selected from the group consisting of ferrous iron, ferric iron, and combinations thereof. In some embodiments, the iron is in any form.

In some embodiments, the portion of the heart is selected from the group consisting of epicardium, myocardium, endocardium, valvular tissue, and a combination thereof. In some embodiments, the portion of the heart is selected from the group consisting of valvular tissue, endocardium, pulmonary valve, brachiocephalic artery, superior vena cava, right pulmonary artery, right pulmonary veins, tricuspid valve, right ventricle, inferior vena cava, descending aorta, papillary muscle, interventricular septum, epicardium, left ventricle, myocardium, mitral valve, aortic valve, left pulmonary veins, left pulmonary artery, aorta, left subclavian artery, pulmonary trunk, left common carotid artery, and a combination thereof. In some embodiments, the portion of the heart is myocardium, or comprises myocardium.

In some embodiments, the change is in the at least one iron deposit. In some embodiments, the change is in at least one selected from the group consisting of an amount, quantity, concentration, shape, size, and pattern of the at least one iron deposit.

In some embodiments, the reference sample is a heart from a control subject, wherein the control subject does not have at least one iron deposit in the heart. In some embodiments, the reference sample is a heart from a control subject, wherein the control subject does not have at least one microbleed in the heart. In some embodiments, the reference sample is the heart from the subject at an earlier point in time.

In some embodiments, the cardiovascular disease is selected from the group consisting of infarcted myocardium, coronary artery disease, coronary heart disease, ischemic heart disease, cardiomyopathy, stroke, hypertensive heart disease, heart failure, pulmonary heart disease, ischemic syndrome, coronary microvascular disease, cardiac dysrhythmias, rheumatic heart disease, aortic aneurysms, cardiomyopathy, atrial fibrillation, congenital heart disease, endocarditis, inflammatory heart disease, endocarditis, inflammatory cardiomegaly, myocarditis, valvular heart disease, cerebrovascular disease, peripheral artery disease, acute stress-induced cardiomyopathy (Takotsubo cardiomyopathy), diffuse myocardial fibrosis, and a combination thereof. In some embodiments, the cardiovascular disease comprises sudden cardiac failure or death, electrical conduction abnormality or mechanical abnormality in myocardium, myocardial inflammation, ventricular arrhythmia, atrial fibrillation, myocardial fibrosis, and/or adverse remodeling of the heart. In some embodiments, the cardiovascular disease comprises myocardial fibrosis. In some embodiments, the cardiovascular disease consists of myocardial fibrosis. In some embodiments, the cardiovascular disease consists essentially of myocardial fibrosis.

In some embodiments, the reference sample is a heart from the subject before the subject is treated for the cardiovascular disease. In some embodiments, the reference sample is a heart from a subject that has been successfully treated for the cardiovascular disease.

In some embodiments, the reference sample is a heart from a control subject, wherein the control subject does not have the cardiovascular disease. In some embodiments, the control subject does not have hypertension, hypertensive crisis, acute hypertensive crisis, or prolonged hypertension. In some embodiments, the reference sample is a heart from a control subject, wherein the control subject has the cardiovascular disease.

In some embodiments, the reference sample is from a heart of a subject that has been successfully treated for hypertension. In some embodiments, the reference sample is from the heart of the subject at an earlier point in time.

In some embodiments, obtaining at least one clinical image, or performing at least one clinical imaging, comprises operating a clinical imaging machine/scanner to obtain the at least one clinical image. In some embodiments, obtaining at least one CT image comprises operating a CT imaging machine/scanner to obtain the at least one CT image. In some embodiments, obtaining at least one SPECT image comprises operating a SPECT imaging machine/scanner to obtain the at least one SPECT image. In some embodiments, obtaining at least one PET image comprises operating a PET imaging machine/scanner to obtain the at least one PET image. In some embodiments, obtaining at least one magnetic resonance image comprises operating a magnetic resonance imaging machine/scanner to obtain the at least one magnetic resonance image.

In some embodiments, the heart tissue is selected from the group consisting of valvular tissue, endocardium, pulmonary valve, brachiocephalic artery, superior vena cava, right pulmonary artery, right pulmonary veins, tricuspid valve, right ventricle, inferior vena cava, descending aorta, papillary muscle, interventricular septum, epicardium, left ventricle, myocardium, mitral valve, aortic valve, left pulmonary veins, left pulmonary artery, aorta, left subclavian artery, pulmonary trunk, left common carotid artery, and a combination thereof.

### BRIEF DESCRIPTION OF THE DRAWINGS

Exemplary embodiments are illustrated in referenced figures. It is intended that the embodiments and figures disclosed herein are to be considered illustrative rather than restrictive.

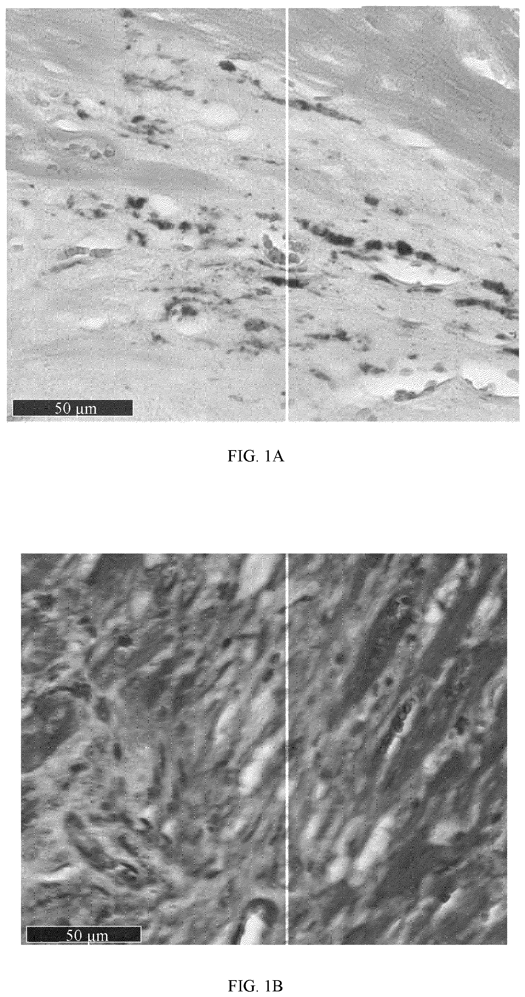

FIG. 1A is a representative Prussian Blue staining image depicting iron in the myocardial tissue, and FIG.1B is a representative elastin Masson's Trichrome staining image depicting the ensuing myocardial fibrosis, both from wild-type rats treated with angiotensin II to induce hypertension.

### DETAILED DESCRIPTION OF THE INVENTION

Unless defined otherwise, technical and scientific terms used herein have the same meaning as commonly understood by one of ordinary skill in the art to which this invention belongs. Allen et al., Remington: The Science and Practice of Pharmacy 22nd ed., Pharmaceutical Press (September 15, 2012); Hornyak et al., Introduction to Nanoscience and Nanotechnology, CRC Press (2008); Smith, March's Advanced Organic Chemistry Reactions, Mechanisms and Structure 7th ed., J. Wiley & Sons (New York, NY 2013); Singleton, Dictionary of DNA and Genome Technology 3rd ed., Wiley-Blackwell (November 28, 2012); and Green and Sambrook, Molecular Cloning: A Laboratory Manual 4th ed., Cold Spring Harbor Laboratory Press (Cold Spring Harbor, NY 2012), provide one skilled in the art with a general guide to many of the terms used in the present application.

One skilled in the art will recognize many methods and materials similar or equivalent to those described herein, which could be used in the practice of the present invention. Other features and advantages of the invention will become apparent from the following detailed description, taken in conjunction with the accompanying drawings, which illustrate, by way of example, various features of embodiments of the invention. Indeed, the present invention is in no way limited to the methods and materials described. For convenience, certain terms employed herein, in the specification, examples and appended claims are collected here.

Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. Unless otherwise defined, all technical and scientific terms used herein have the same meaning as commonly understood by one of ordinary skill in the art to which this invention belongs. It should be understood that this invention is not limited to the particular methodology, protocols, and reagents, etc., described herein and as such can vary. The definitions and terminology used herein are provided to aid in describing particular embodiments, and are not intended to limit the claimed invention, because the scope of the invention is limited only by the claims.

The term "comprising" or "comprises" is used in reference to compositions, methods, systems, articles of manufacture, and respective component(s) thereof, that are useful to an embodiment, yet open to the inclusion of unspecified elements, whether useful or not. It will be understood by those within the art that, in general, terms used herein are generally intended as "open" terms (e.g., the term "including" should be interpreted as "including but not limited to," the term "having" should be interpreted as "having at least," the term "includes" should be interpreted as "includes but is not limited to," etc.). Although the open-ended term "comprising," as a synonym of terms such as including, containing, or having, is used herein to describe and claim the invention, the present invention, or embodiments thereof, may alternatively be described using alternative terms such as "consisting of" or "consisting essentially of."

Unless stated otherwise, the terms "a" and "an" and "the" and similar references used in the context of describing a particular embodiment of the application (especially in the context of claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (for example, "such as") provided with respect to certain embodiments herein is intended merely to better illuminate the application and does not pose a limitation on the scope of the application otherwise claimed. The abbreviation, "e.g." is derived from the Latin exempli gratia, and is used herein to indicate a non-limiting example. Thus, the abbreviation "e.g." is synonymous with the term "for example." No language in the specification should be construed as indicating any non-claimed element essential to the practice of the application.

"Optional" or "optionally" means that the subsequently described circumstance may or may not occur, so that the description includes instances where the circumstance occurs and instances where it does not.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

The terms "treat," "treatment," "treating," or "amelioration" when used in reference to a disease, disorder or medical condition, refer to both therapeutic treatment and prophylactic or preventative measures, wherein the object is to reverse, alleviate, ameliorate, inhibit, lessen, slow down or stop the progression or severity of a symptom or condition. The term "treating" includes reducing or alleviating at least one adverse effect or symptom of a condition. Treatment is generally "effective" if one or more symptoms or clinical markers are reduced. Alternatively, treatment is "effective" if the progression of a disease, disorder or medical condition is reduced or halted. That is, "treatment" includes not just the improvement of symptoms or markers, but also a cessation or at least slowing of progress or worsening of symptoms that would be expected in the absence of treatment. Also, "treatment" may mean to pursue or obtain beneficial results, or lower the chances of the individual developing the condition even if the treatment is ultimately unsuccessful. Those in need of treatment include those already with the condition as well as those prone to have the condition or those in whom the condition is to be prevented. Non-limiting examples of treatments or therapeutic treatments include at least one selected from pharmacological therapies, biological therapies, interventional surgical treatments, and combinations thereof. Non-limiting examples of therapeutic treatments include any one or more of coronary revascularization through stenting, coronary bypass grafting, or medical therapy, or combinations thereof. Non-limiting examples of medical therapies (blood pressure medications) include statins, LDL lowering, beta blockers, angiotension-converting enzyme (ACE) inhibitors, calcium channel blockers, angiotension II antagonists, vasodilators, diuretics, renin inhibitors, aspirin, etc. or combinations thereof.

The term "preventative treatment" means maintaining or improving a healthy state or non-diseased state of a healthy subject or subject that does not have a disease. The term "preventative treatment" or "prophylaxis" also means to prevent or to slow the appearance of symptoms associated with a condition, disease, or disorder. The term "preventative treatment" also means to prevent or slow a subject from obtaining a condition, disease, or disorder.

"Beneficial results" or "desired results" may include, but are in no way limited to, lessening or alleviating the severity of the disease condition, preventing the disease condition from worsening, curing the disease condition, preventing the disease condition from developing, lowering the chances of a patient developing the disease condition, decreasing morbidity and mortality, and prolonging a patient's life or life expectancy. As non-limiting examples, "beneficial results" or "desired results" may be alleviation of one or more symptom(s), diminishment of extent of the deficit, stabilized (i.e., not worsening) state of a cardiovascular disease, delay or slowing of a cardiovascular disease, and amelioration or palliation of symptoms associated with a cardiovascular disease.

The term "disease" refers to an abnormal condition affecting the body of an organism. The term "disorder" refers to a functional abnormality or disturbance. The terms disease or disorder are used interchangeably herein unless otherwise noted or clear given the context in which the term is used.

"Diseases", "conditions" and "disease conditions," as used herein may include, but are in no way limited to any form of cardiovascular conditions, diseases or disorders. Cardiovascular diseases are a class of diseases that involve the heart or blood vessels. Non-limiting examples of cardiovascular disease include: coronary artery disease, coronary heart disease, ischemic heart disease (IHD), cardiomyopathy, stroke, hypertensive heart disease, heart failure, pulmonary heart disease, ischemic syndrome, coronary microvascular disease, cardiac dysrhythmias, rheumatic heart disease (RHD), aortic aneurysms, cardiomyopathy, atrial fibrillation, congenital heart disease, endocarditis, inflammatory heart disease, endocarditis, inflammatory cardiomegaly, myocarditis, valvular heart disease, cerebrovascular disease, and peripheral artery disease (PAD).

A "healthy subject" or "normal subject" is a subject that does not have a disease or disorder. In some embodiments, a healthy subject or a normal subject does not have hypertension or hypertensive crisis.

The term "administering," refers to the placement an agent as disclosed herein into a subject by a method or route which results in at least partial localization of the agents at a desired site. "Route of administration" may refer to any administration pathway known in the art, including but not limited to aerosol, nasal, via inhalation, oral, anal, intra-anal, peri-anal, transmucosal, transdermal, parenteral, enteral, topical or local. "Parenteral" refers to a route of administration that is generally associated with injection, including intratumoral, intracranial, intraventricular, intrathecal, epidural, intradural, intraorbital, infusion, intracapsular, intracardiac, intradermal, intramuscular, intraperitoneal, intrapulmonary, intraspinal, intrasternal, intrathecal, intrauterine, intravascular, intravenous, intraarterial, subarachnoid, subcapsular, subcutaneous, transmucosal, or transtracheal. Via the parenteral route, the compositions may be in the form of solutions or suspensions for infusion or for injection, or as lyophilized powders. Via the enteral route, the pharmaceutical compositions can be in the form of tablets, gel capsules, sugar-coated tablets, syrups, suspensions, solutions, powders, granules, emulsions, microspheres or nanospheres or lipid vesicles or polymer vesicles allowing controlled release. Via the topical route, the pharmaceutical compositions can be in the form of aerosol, lotion, cream, gel, ointment, suspensions, solutions or emulsions. In accordance with the present invention, "administering" can be self-administering. For example, it is considered as "administering" that a subject consumes a composition as disclosed herein.

"Diagnostic" means identifying the presence or nature of a pathologic condition, disease, or disorder and includes identifying patients who are at risk of developing a specific condition, disease or disorder. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, a disease, or a disorder, it suffices if the method provides a positive indication that aids in diagnosis.

By "at risk of" is intended to mean at increased risk of, compared to a normal subject, or compared to a control group, e.g. a patient population. Thus a subject carrying a particular marker may have an increased risk for a specific condition, disease or disorder, and be identified as needing further testing. "Increased risk" or "elevated risk" mean any statistically significant increase in the probability, e.g., that the subject has the disorder. In some embodiments the risk is increased by at least 10% over the control group with which the comparison is being made. In some embodiments, the risk is increased by at least 20% over the control group with which the comparison is being made. In some embodiments, the risk is increased by at least 50% over the control group with which the comparison is being made.

The terms "detection", "detecting" and the like, may be used in the context of detecting a condition, detecting a disease or a disorder (e.g. when positive assay results are obtained).

The term "diagnosis," or "dx," refers to the identification of the nature and cause of a certain phenomenon. A diagnosis typically refers to a medical diagnosis, which is the process of determining which disease or condition explains a symptoms and signs. A diagnostic procedure, often a diagnostic test or assay, can be used to provide a diagnosis.

The term "prognosis," or "px," refers to predicting the likely outcome of a current standing. For example, a prognosis can include the expected duration and course of a disease or disorder, such as progressive decline or expected recovery.

The term "theranostics," or "tx" refers to a diagnosis or prognosis used in the context of a medical treatment. For example, theranostics can include diagnostic testing used for selecting appropriate and optimal therapies (or the inverse) based on the context of genetic content or other molecular or cellular analysis. Theranostics includes pharmacogenomics, personalized and precision medicine.

"Sample" is used herein in its broadest sense. The term "biological sample" denotes a sample taken or isolated from a biological organism. Exemplary biological samples include, but are not limited to, cheek swab; mucus; whole blood, blood, serum; plasma; urine; saliva; semen; lymph; fecal extract; sputum; other body fluid or biofluid; cell sample; and tissue sample etc. The term also includes a mixture of the above-mentioned samples. The term "sample" also includes untreated or pretreated (or pre-processed) biological samples. In some embodiments, a sample can comprise one or more cells from the subject. In some embodiments, a sample is a tissue or tissue sample. In some embodiments, a sample is a heart. In some embodiments, a sample is at least one portion of a heart. In some embodiments, a sample is at least one heart tissue. In some embodiments, the sample (e.g., heart) is examined in a non-invasive manner, hence the sample is (within) a living subject.

A "subject" means a human or animal. Usually the animal is a vertebrate such as a primate, rodent, domestic animal or game animal. Primates include chimpanzees, cynomologous monkeys, spider monkeys, and macaques, e.g., Rhesus. Rodents include mice, rats, woodchucks, ferrets, rabbits and hamsters. Domestic and game animals include cows, horses, pigs, deer, bison, buffalo, feline species, e.g., domestic cat, and canine species, e.g., dog, fox, wolf. The terms, "patient", "individual" and "subject" are used interchangeably herein. In an embodiment, the subject is mammal. The mammal can be a human, non-human primate, mouse, rat, dog, cat, horse, or cow, but are not limited to these examples. In addition, the methods described herein can be used to treat domesticated animals and/or pets. In some embodiments, the subject is a human.

"Mammal" refers to any member of the class Mammalia, including, without limitation, humans and nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be included within the scope of this term.

A subject can be one who has been previously diagnosed with or identified as suffering from or having a condition in need of treatment (e.g., a microbleed, a cardiovascular disease; or hypertension, hypertensive crisis) or one or more complications related to the condition, and optionally, have already undergone treatment for the condition or the one or more complications related to the condition. Alternatively, a subject can also be one who has not been previously diagnosed as having a condition or one or more complications related to the condition. For example, a subject can be one who exhibits one or more risk factors for a condition or one or more complications related to the condition or a subject who does not exhibit risk factors. A "subject in need" of treatment for a particular condition can be a subject suspected of having that condition, diagnosed as having that condition, already treated or being treated for that condition, not treated for that condition, or at risk of developing that condition.

"Microbleeds" refer to small hemorrhages which are likely caused by structural abnormalities of the small vessels in an organ. In some embodiments, microbleeds refer to conditions where red blood cells are found outside the vasculature, which may be due to abnormal permeability of the vasculature or rupture of a blood vessel. Microbleeds occur adjacent to small vessels commonly affected by amyloid angiopathy or hypertensive arteriopathy. In some instances, a subject can have prolonged microbleeds; and in other instances, microbleeds are acute microbleeds. In some embodiments, the methods disclosed herein are directed to treatment, prophylaxis, assessment, and/or diagnosis of myocardial microbleeds, especially in a subject with hypertension. In some embodiments, the methods disclosed herein are directed to treatment, prophylaxis, assessment, and/or diagnosis of microbleeds except for cerebral microbleeds. In some embodiments, the methods herein are directed to myocardium or the heart tissue, and not the brain. In some embodiments, the microbleeds in the methods disclosed herein are new or secondary microbleeds in the myocardium or another tissue that occur following an ischemic stroke.

Symptoms associated with a microbleed, especially an acute microbleed, can include a sudden tingling sensation (e.g., in a side or both sides of face, arms, chest, limbs), heaviness of the arm and/or legs, double vision and/or nausea.

"Hypertension," also called high blood pressure, refers to blood pressure that is higher than normal (especially on repeated measurements over time), where a normal blood pressure level is less than 120/80 mm Hg. In some embodiments, a subject with systolic blood pressure of 130 mm Hg or greater has high blood pressure. In some embodiments, a subject with high blood pressure also has diastolic blood pressure of 80 mm Hg or greater.

In various embodiments, a subject is an adult human being, and he or she is diagnosed with hypertension if his or her blood pressure is 130/80 mm Hg or higher. In some embodiments, health care professionals diagnose patients with high blood pressure if the patients' blood pressure is consistently 130/80 mm Hg or higher, according to the American College of Cardiology/American Heart Association Guideline for the Prevention, Detection, Evaluation, and Management of High Blood Pressure in Adults (2017 Guideline). According to the 2017 Guideline, patients are diagnosed to have elevated blood pressure if they have a systolic blood pressure of 120-129 mm Hg and a diastolic blood pressure of less than 80 mm Hg. In some embodiments, health care professionals diagnose patients with high blood pressure if the patients' blood pressure is consistently 140/90 mm Hg or higher, according to the Seventh Report of the Joint National Committee on Prevention, Detection, Evaluation, and Treatment of High Blood Pressure (2003 Guideline). According to the 2003 Guideline, patients are diagnosed to be at risk or prehypertension if they have a systolic blood pressure between 120 and 139 mm Hg and a diastolic blood pressure between 80 and 89 mm Hg.

"Hypertensive crisis" generally refers to an acute event that transiently increases blood pressure due to emotional distress. An example of hypertensive crisis includes Takotsubo cardiomyopathy (also referred to as stress cardiomyopathy).

In some embodiments, a subject is a child or adolescent human being, and he or she is diagnosed with hypertension when their average blood pressure is at or above the 95th percentile for their age, sex and height when measured multiple times over three visits or more.

"Ferroptosis" refers to an iron-dependent form of cell death. The small molecule erastin inhibits the import of cysteine, leading to glutathione depletion and inactivation of phospholipid peroxidase, glutathione peroxidase 4 (GPX4). GPX4 converts potentially toxic lipid hydroperoxides to non-toxic lipid alcohols. Inactivation of GPX4 through depletion of GSH with erastin, or with a direct GPX4 inhibitor (1*S*, 3*R*)-RSL3, ultimatedly results in overwhelming lipid peroxidation that causes cell death. Iron is required for the accumulation of lipid peroxides and the execution of ferroptosis. Genes involved in regulating ferroptosis, or to serve as markers of ferroptosis, include GXP4, acyl-CoA synthetase long-chain family member 4 (ACSL-4), aldo-keto reductase family 1 member C1 (AKR1C1-3), lipoxygenase, ATP synthase H+transporting mitochondrial Fo complex subunit C3 (ATP5G3), cystathionine beta synthase (CBS), CD44 molecule, ChaC Glutathione specific gamma-glutamylcyclotransferase 1 (CHAC1), CDGSH iron sulfur domain 1 (CISD1), citrate synthase (CS), dipeptidyl-dippeptidase-4 (DPP4), Fanconi anemia complementation group D2 (FANCD2), glutamate-cysteine ligase (GCLC/GCLM), glutaminase 2 (GLS2), glutathione synthetase (GSS), 3-hydroxy-3-methylglutaryl-CoA reductase (HMGCR), heat shock protein beta 1/5 (HSPB1/5), kiss of death (KOD), lysophosphatidylcholine acyltransferase 3 (LPCAT3), metallothionein-1G (MT1G), nuclear receptor coactivator 4 (NCOA4), nuclear factor erythroid 2 like 2 (NFE2L2), prostaglandin-endoperoxide synthase 2 (PTGS2), ribosomal protein L8 (RPL8), spermidine/spermine N1-acetyltransferase 1 (SAT2), solute carrier family 7 member 11 (SLC7A11), squalene synthase (SQS), transferrin receptor (TFRC), tumor protein 53 (TP53), and ER membrane protein complex subunit 2 (TT35/EMC2). In some embodiments, a ferroptosis marker in the methods disclosed herein includes any one, two, three, four, five, six, seven, eight, nine, 10, 11, 12, 13, 14, or 15 of the genes that are involved in regulating ferroptosis, and can be measured by way of quantifying its or their DNA amounts, mRNA amounts, cDNA amounts, or protein expression levels.

In various embodiments, the treatment may be provided as pharmaceutical compositions. In various embodiments, the pharmaceutical compositions according to the invention may be formulated for delivery via a route of administration. In certain embodiments, the pharmaceutical compositions are formulated for intravascular, intravenous, or intraarterial administration. In one embodiment, the pharmaceutical compositions are formulated for intravenous administration as a single bolus.

In various embodiments, the pharmaceutical compositions can contain any pharmaceutically acceptable excipient. "Pharmaceutically acceptable excipient" means an excipient that is useful in preparing a pharmaceutical composition that is generally safe, non-toxic, and desirable, and includes excipients that are acceptable for veterinary use as well as for human pharmaceutical use. Such excipients may be solid, liquid, semisolid, or, in the case of an aerosol composition, gaseous. Examples of excipients include but are not limited to starches, sugars, microcrystalline cellulose, diluents, granulating agents, lubricants, binders, disintegrating agents, wetting agents, emulsifiers, coloring agents, release agents, coating agents, sweetening agents, flavoring agents, perfuming agents, preservatives, antioxidants, plasticizers, gelling agents, thickeners, hardeners, setting agents, suspending agents, surfactants, humectants, carriers, stabilizers, and combinations thereof.

In various embodiments, the pharmaceutical compositions according to the invention can contain any pharmaceutically acceptable carrier. "Pharmaceutically acceptable carrier" as used herein refers to a pharmaceutically acceptable material, composition, or vehicle that is involved in carrying or transporting a compound of interest from one tissue, organ, or portion of the body to another tissue, organ, or portion of the body. For example, the carrier may be a liquid or solid filler, diluent, excipient, solvent, or encapsulating material, or a combination thereof. Each component of the carrier must be "pharmaceutically acceptable" in that it must be compatible with the other ingredients of the formulation. It must also be suitable for use in contact with any tissues or organs with which it may come in contact, meaning that it must not carry a risk of toxicity, irritation, allergic response, immunogenicity, or any other complication that excessively outweighs its imaging benefits.

The pharmaceutical compositions according to the invention can also be encapsulated, tableted or prepared in an emulsion or syrup for oral administration. Pharmaceutically acceptable solid or liquid carriers may be added to enhance or stabilize the composition, or to facilitate preparation of the composition. Liquid carriers include syrup, peanut oil, olive oil, glycerin, saline, alcohols and water. Solid carriers include starch, lactose, calcium sulfate, dihydrate, terra alba, magnesium stearate or stearic acid, talc, pectin, acacia, agar or gelatin. The carrier may also include a sustained release material such as glyceryl monostearate or glyceryl distearate, alone or with a wax.

The pharmaceutical compositions are made following the conventional techniques of pharmacy involving dry milling, mixing, and blending for powder forms; milling, mixing, granulation, and compressing, when necessary, for tablet forms; or milling, mixing and filling for hard gelatin capsule forms. When a liquid carrier is used, the preparation will be in the form of a syrup, elixir, emulsion or an aqueous or non-aqueous suspension. Such a liquid formulation may be administered directly p.o. or filled into a soft gelatin capsule.

The pharmaceutical compositions may be delivered in an effective amount for reducing or removing iron deposits from the heart, which can be accessed for efficacy via cardiac magnetic resonance imaging. The precise effective amount is that amount of the composition that will yield the most effective results in terms of removing iron deposits and improving heart function and/or remodeling. This amount will vary depending upon a variety of factors, including but not limited to the characteristics of the treatment (including activity, pharmacokinetics, pharmacodynamics, and bioavailability), the physiological condition of the subject (including age, sex, disease type and stage, general physical condition, responsiveness to a given dosage, and type of medication), the nature of the pharmaceutically acceptable carrier or carriers in the formulation, and the route of administration. One skilled in the clinical and pharmacological arts will be able to determine a stress-imaging effective amount through routine experimentation, for instance, by monitoring image quality and adjusting the dosage accordingly.

Before administration to patients, formulants may be added to the pharmaceutical composition. In some embodiments, the formulant is a liquid formulant. For example, these formulants may include oils, polymers, vitamins, carbohydrates, amino acids, salts, buffers, albumin, surfactants, bulking agents or combinations thereof.

Carbohydrate formulants include sugar or sugar alcohols such as monosaccharides, disaccharides, or polysaccharides, or water soluble glucans. The saccharides or glucans can include fructose, dextrose, lactose, glucose, mannose, sorbose, xylose, maltose, sucrose, dextran, pullulan, dextrin, alpha and beta cyclodextrin, soluble starch, hydroxethyl starch and carboxymethylcellulose, or mixtures thereof. "Sugar alcohol" is defined as a C4 to C8 hydrocarbon having an -OH group and includes galactitol, inositol, mannitol, xylitol, sorbitol, glycerol, and arabitol. These sugars or sugar alcohols mentioned above may be used individually or in combination. There is no fixed limit to amount used as long as the sugar or sugar alcohol is soluble in the aqueous preparation. In some embodiments, the sugar or sugar alcohol concentration is between 1.0 w/v % and 7.0 w/v %. In some embodiments, the sugar or sugar alcohol concentration is between 2.0 and 6.0 w/v %.

Amino acids formulants include levorotary (L) forms of carnitine, arginine, and betaine; however, other amino acids may be added.

Polymers formulants include polyvinylpyrrolidone (PVP) with an average molecular weight between 2,000 and 3,000, or polyethylene glycol (PEG) with an average molecular weight between 3,000 and 5,000.

A buffer may also be used in the pharmaceutical compositions to minimize pH changes in the solution before lyophilization or after reconstitution. Most any physiological buffer may be used including but not limited to citrate, phosphate, succinate, and glutamate buffers or mixtures thereof. In some embodiments, the concentration is from 0.01 to 0.3 molar. Surfactants that can be added to the formulation are shown in EP Nos. 270,799 and 268,110.

Another drug delivery system for increasing circulatory half-life is the liposome. Methods of preparing liposome delivery systems are discussed in Gabizon et al., Cancer Research (1982) 42:4734; Cafiso, Biochem Biophys Acta (1981) 649:129; and Szoka, Ann Rev Biophys Eng (1980) 9:467. Other drug delivery systems are known in the art and are described in, e.g., Poznansky et al., DRUG DELIVERY SYSTEMS (R. L. Juliano, ed., Oxford, N.Y. 1980), pp. 253-315; M. L. Poznansky, Pharm Revs (1984) 36:277.

After the liquid pharmaceutical composition is prepared, it may be lyophilized to prevent degradation and to preserve sterility. Methods for lyophilizing liquid compositions are known to those of ordinary skill in the art. Just prior to use, the composition may be reconstituted with a sterile diluent (Ringer's solution, distilled water, or sterile saline, for example) which may include additional ingredients. Upon reconstitution, the composition is administered to subjects using those methods that are known to those skilled in the art.

The pharmaceutical compositions of the invention may be sterilized by conventional, well-known sterilization techniques. The resulting solutions may be packaged for use or filtered under aseptic conditions and lyophilized, the lyophilized preparation being combined with a sterile solution prior to administration. The compositions may contain pharmaceutically-acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, and stabilizers (e.g., 1-20% maltose, etc.).

Many variations and alternative elements have been disclosed in embodiments of the present invention. Still further variations and alternate elements will be apparent to one of skill in the art. Among these variations, without limitation, is the selection of steps, pharmaceutical compositions, administration routes and devices, imaging technologies for the methods, and the diseases and other clinical conditions that may be diagnosed, prognosed or treated therewith. Various embodiments of the invention can specifically include or exclude any of these variations or elements.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements.

Many people suffer from hypertension or are at risk of developing hypertension. Hypertension can lead to a various adverse outcomes including cardiovascular disease, cardiac arrhythmia, sudden cardiac death, and death. How hypertension leads to heat failure or other major adverse cardiovascular events is not known.

We have discovered the occurrence of microbleeds, detected as iron deposits, in the hearts of subjects with hypertension. Specifically, we have discovered the occurrence of microbleeds, detected as iron deposits, within the myocardium in spontaneously hypertensive rats. This is an important finding because the presence of iron within the myocardium may be a primary mediator of adverse cardiac remodeling and fatal outcomes in patients with hypertension or hypertensive crisis. Our finding that microbleeds take place in hypertensive hearts resulting in iron deposition in the myocardium may help provide an understanding of the pathophysiology of cardiac remodeling in hypertension.

Therefore, a non-limiting object of the present invention is to provide methods for detecting, diagnosing, and/or treating microbleeds in the heart so as to reduce adverse outcomes in people with hypertension.

### Various Non-Limiting Embodiments of the Invention

In various embodiments, the methods include a heart of a subject. In some embodiments, the heart is a human heart. In some embodiments, the subject is a human.

In various embodiments, the methods include at least one portion of a heart of a subject.

In various embodiments, the methods include at least one heart tissue of a subject. In some embodiments, the heart tissue is human heart tissue. In some embodiments, the subject is a human.

In various embodiments, the methods include a heart of a control subject. In some embodiments, the heart is a human heart. In some embodiments, the control subject is a human.

In various embodiments, the methods include at least one portion of a heart of a control subject. In some embodiments, the heart is a human heart. In some embodiments, the control subject is a human.

In various embodiments, the methods include at least one heart tissue of a control subject. In some embodiments, the heart tissue is human heart tissue. In some embodiments, the control subject is a human.

In various embodiments, methods are provided for identifying and/or assessing at least one microbleed in a heart of a subject, comprising: obtaining at least one clinical image (e.g., SPECT, PET, CT, ultrasound, or magnetic resonance image) of the heart of the subject; and detecting at least one iron deposit in the heart of the subject from the at least one clinical image, wherein detection of the iron deposit in the heart of the subject is indicative of the at least one microbleed in the heart of the subject. In various embodiments, the subject has or is diagnosed with hypertension. In some embodiments, detecting at least one iron deposit in the heart of the subject identifies that the subject has had myocardial microbleeding. In further embodiments, detecting at least one iron deposit in the heart of the subject indicates that the subject has hypertension. In further embodiments, the subject identified as having myocardial microbleeds have a higher content of fat cells infiltration compared to a reference sample, especially if the subject has had the myocardial microbleeds or hypertension for at least 1 month, 2 months, 3 months, 4 months, 5 months, 6 months, 1 year, 2 years, 3 years, 5 years or longer.

In various embodiments, methods are provided for detecting at least one microbleed in a heart of a subject, comprising: obtaining at least one clinical image (e.g., SPECT, PET, CT, ultrasound, or magnetic resonance image) of the heart of the subject; and detecting at least one iron deposit in the heart of the subject from the at least one clinical image, wherein detection of the at least one iron deposit in the heart of the subject is indicative of and/or is a detection of the at least one microbleed in the heart of the subject.

In various embodiments, methods are provided for prognosing at least one microbleed in a heart of a subject, comprising: obtaining at least one clinical image of the heart of the subject; and detecting at least one iron deposit in the heart of the subject from the at least one clinical image, wherein detection of the at least one iron deposit in the heart of the subject is a prognosis of the at least one microbleed in the heart of the subject.

In various embodiments, methods are provided for diagnosing at least one microbleed in a heart of a subject, comprising: obtaining at least one clinical image of the heart of the subject; and detecting at least one iron deposit in the heart of the subject from the at least one clinical image, wherein detection of the at least one iron deposit in the heart of the subject is a diagnosis of the at least one microbleed in the heart of the subject.

In various embodiments, methods are provided for assessing and/or determining the risk of developing at least one microbleed in a heart of a subject, comprising: obtaining at least one clinical image of the heart of the subject; and detecting at least one iron deposit in the heart of the subject from the at least one image, wherein detection of the at least one iron deposit in the heart of the subject is indicative of an increased risk of developing at least one microbleed in the heart of the subject.

In various embodiments, methods are provided for identifying and/or assessing at least one microbleed in at least one portion of a heart of a subject, comprising: obtaining at least one clinical image of the at least one portion of the heart of the subject; and detecting at least one iron deposit in the at least one portion of the heart of the subject from the at least one clinical image, wherein detection of the at least one iron deposit in the at least one portion of the heart of the subject is indicative of the at least one microbleed in the at least one portion of the heart of the subject.

In various embodiments, methods are provided for detecting at least one microbleed in at least one portion of a heart of a subject, comprising: obtaining at least one clinical image of the at least one portion of the heart of the subject; and detecting at least one iron deposit in the at least one portion of the heart of the subject from the at least one clinical image, wherein detection of the at least one iron deposit in the at least one portion of the heart of the subject is indicative of and/or is a detection of the at least one microbleed in the at least one portion of the heart of the subject.

In various embodiments, methods are provided for prognosing at least one microbleed in at least one portion of a heart of a subject, comprising: obtaining at least one clinical image of the at least one portion of the heart of the subject; and detecting at least one iron deposit in the at least one portion of the heart of the subject from the at least one clinical image, wherein detection of the at least one iron deposit in the at least one portion of the heart of the subject is a prognosis of the at least one microbleed in the at least one portion of the heart of the subject.

In various embodiments, methods are provided for diagnosing at least one microbleed in at least one portion of a heart of a subject, comprising: obtaining at least one clinical image of the at least one portion of the heart of the subject; and detecting at least one iron deposit in the at least one portion of the heart of the subject from the at least one clinical image, wherein detection of the at least one iron deposit in the at least one portion of the heart of the subject is a diagnosis of the at least one microbleed in the at least one portion of the heart of the subject.

In various embodiments, methods are provided for assessing and/or determining the risk of developing at least one microbleed in at least one portion of a heart of a subject, comprising: obtaining at least one clinical image of the at least one portion of the heart of the subject; and detecting at least one iron deposit in the at least one portion of the heart of the subject from the at least one clinical image, wherein detection of the at least one iron deposit in the at least one portion of the heart of the subject is indicative of an increased risk of the subject developing at least one microbleed in the at least one portion of the heart of the subject.

In various embodiments, methods are provided for identifying and/or assessing at least one microbleed in at least one heart tissue of a subject, comprising: obtaining at least one clinical image of the at least one heart tissue; and detecting at least one iron deposit in the at least one heart tissue from the at least one image, wherein detection of the at least one iron deposit in the at least one heart tissue is indicative of the at least one microbleed in the at least one heart tissue of the subject.

In various embodiments, methods are provided for detecting at least one microbleed in at least one heart tissue of a subject, comprising: obtaining at least one clinical image of the at least one heart tissue; and detecting at least one iron deposit in the at least one heart tissue from the at least one clinical image, wherein detection of the at least one iron deposit in the at least one heart tissue is indicative of and/or is a detection of the at least one microbleed in the at least one heart tissue of the subject.

In various embodiments, methods are provided for prognosing at least one microbleed in at least one heart tissue of a subject, comprising: obtaining at least one clinical image of the heart; and detecting at least one iron deposit in the heart from the at least one clinical image, wherein detection of the at least one iron deposit in the heart is a prognosis of the at least one microbleed in the heart of the subject.

In various embodiments, methods are provided for diagnosing at least one microbleed in at least one heart tissue of a subject, comprising: obtaining at least one clinical image of the at least one heart tissue; and detecting at least one iron deposit in the at least one heart tissue from the at least one clinical image, wherein detection of the at least one iron deposit in the at least one heart tissue is a diagnosis of the at least one microbleed in the at least one heart tissue of the subject.

In various embodiments, methods are provided for assessing and/or determining the risk of developing at least one microbleed in at least one heart tissue of a subject, comprising: obtaining at least one clinical image of the at least one heart tissue; and detecting at least one iron deposit in the at least one heart tissue from the at least one clinical image, wherein detection of the at least one iron deposit in the at least one heart tissue is indicative of an increased risk of the subject developing at least one microbleed in the at least one heart tissue of the subject.

In various embodiments, methods are provided for identifying and/or assessing at least one microbleed in a heart of a subject, comprising: obtaining at least one clinical image of the heart of the subject; and detecting and/or measuring and/or quantifying the presence of at least one iron deposit in the heart of the subject from the at least one clinical image of the heart of the subject; and comparing the clinical image of the heart of the subject to at least one clinical image from a reference sample, wherein the presence of at least one iron deposit in the heart of the subject relative to the reference sample is indicative of at least one microbleed in the heart of the subject.

In various embodiments, methods are provided for detecting at least one microbleed in a heart of a subject, comprising: obtaining at least one clinical image of the heart of the subject; and detecting and/or measuring and/or quantifying the presence of at least one iron deposit in the heart of the subject from the at least one clinical image of the heart of the subject; and comparing the clinical image of the heart of the subject to at least one clinical image from a reference sample, wherein the presence of at least one iron deposit in the heart of the subject relative to the reference sample is indicative of at least one microbleed in the heart of the subject.

In various embodiments, methods are provided for prognosing at least one microbleed in a heart of a subject, comprising: obtaining at least one clinical image of the heart of the subject; and detecting and/or measuring and/or quantifying at least one iron deposit in the heart of the subject from the at least one clinical image of the heart of the subject; and comparing the clinical image of the heart of the subject to at least one clinical image from a reference sample, wherein the presence of at least one iron deposit in the heart of the subject relative to the reference sample is a prognosis of at least one microbleed in the heart of the subject.

In various embodiments, methods are provided for diagnosing at least one microbleed in a heart of a subject, comprising: obtaining at least one clinical image of the heart of the subject; and detecting and/or measuring and/or quantifying at least one iron deposit in the heart of the subject from the at least one clinical image of the heart of the subject; and comparing the clinical image of the heart of the subject to at least one clinical image from a reference sample, wherein the presence of at least one iron deposit in the heart of the subject relative to the reference sample is a diagnosis of at least one microbleed in the heart of the subject.

In various embodiments, methods are provided for assessing and/or determining the risk of developing at least one microbleed in a heart of a subject, comprising: obtaining at least one clinical image of the heart of the subject; and detecting and/or measuring and/or quantifying at least one iron deposit in the heart from the at least one clinical image of the heart of the subject; and comparing the clinical image of the heart of the subject to at least one clinical image from a reference sample, wherein the presence of at least one iron deposit in the heart of the subject relative to the reference sample is indicative of an increased risk of the subject developing at least one microbleed in the heart of the subject.

In various embodiments, methods are provided for determining progression of at least one microbleed in a heart of a subject, comprising: obtaining at least one clinical image of the heart of the subject; and detecting and/or measuring and/or quantifying at least one iron deposit in the heart of the subject from the at least one clinical image of the heart of the subject; and comparing the clinical image of the heart of the subject to at least one clinical image from a reference sample, wherein a change in the clinical image of the heart of the subject relative to the at least one clinical image from the reference sample is indicative of progression of at least one microbleed in the heart of the subject.

In various embodiments, methods are provided for identifying and/or assessing cardiovascular disease in a subject, comprising: obtaining at least one clinical image of a heart of the subject; detecting at least one iron deposit in the heart of the subject from the at least one clinical image of the heart of the subject, wherein detection of the iron deposit in the heart of the subject is indicative of a presence at least one microbleed in the heart of the subject; comparing the clinical image of heart of the subject to at least one clinical image from a reference sample, wherein a change in the clinical image of heart of the subject relative to the at least one clinical image from the reference sample is indicative of cardiovascular disease in the subject.

In various embodiments, methods are provided for detecting cardiovascular disease in a subject, comprising: obtaining at least one clinical image of a heart of the subject; detecting at least one iron deposit in the heart of the subject from the at least one clinical image of the heart of the subject, wherein detection of the at least one iron deposit in the heart of the subject is indicative of the presence at least one microbleed in the heart of the subject; and comparing the at least one clinical image of the heart of the subject to at least one clinical image from a reference sample, wherein a change in the at least one clinical image of heart of the subject relative to the at least one clinical image from the reference sample is indicative of cardiovascular disease in the subject.

In various embodiments, methods are provided for detecting cardiovascular disease in a subject, comprising: obtaining at least one clinical image of a heart of the subject; detecting at least one iron deposit in the heart of the subject from the at least one clinical image of the heart of the subject, wherein detection of the at least one iron deposit in the heart of the subject is indicative of the presence at least one microbleed in the heart of the subject; comparing the at least one clinical image of heart of the subject to at least one clinical image from a reference sample, wherein a change in the at least one clinical image of heart of the subject relative to the at least one clinical image from the reference sample is indicative of cardiovascular disease in the subject.

In various embodiments, methods are provided for prognosing cardiovascular disease in a subject, comprising: obtaining at least one clinical image of the subject's heart; detecting at least one iron deposit in the subject's heart from the at least one clinical image of the heart of the subject, wherein detection of the iron deposit in the subject's heart is indicative of the presence at least one microbleed in the subject's heart; and comparing the clinical image of subject's heart to at least one clinical image from a reference sample, wherein a change in the clinical image of subject's heart relative to the at least one clinical image from the reference sample is a prognosis of cardiovascular disease in the subject. In some embodiments, subjects with myocardial microbleeds have a poorer heart remodeling outcome (e.g., in terms of poorer heart function) or longer remodeling time period, compared to a control subjects.

In various embodiments, methods are provided for diagnosing cardiovascular disease in a subject, comprising: obtaining at least one clinical image of the subject's heart; detecting at least one iron deposit in the subject's heart from the at least one clinical image of the heart of the subject, wherein detection of the iron deposit in the subject's heart is indicative of the presence at least one microbleed in the subject's heart; and comparing the clinical image of subject's heart to at least one clinical image from a reference sample, wherein a change in the clinical image of subject's heart relative to the at least one clinical image from the reference sample is a diagnosis of cardiovascular disease in the subject.

In various embodiments, methods are provided for assessing and/or determining the risk of developing cardiovascular disease in a subject, comprising: obtaining at least one clinical image of the subject's heart; detecting at least one iron deposit in the subject's heart from the at least one clinical image of the heart of the subject, wherein detection of the iron deposit in the subject's heart is indicative of the presence at least one microbleed in the subject's heart; and comparing the clinical image of subject's heart to at least one clinical image from a reference sample, wherein a change in the clinical image of subject's heart relative to the at least one clinical image from the reference sample is indicative of an increased risk of the subject developing cardiovascular disease in the subject.

In various embodiments, methods are provided for determining progression of cardiovascular disease in a subject, comprising: obtaining at least one clinical image of the subject's heart; detecting at least one iron deposit in the subject's heart from the at least one clinical image of the heart of the subject, wherein detection of the iron deposit in the subject's heart is indicative of the presence at least one microbleed in the subject's heart; and comparing the clinical image of subject's heart to at least one clinical image from a reference sample, wherein a change in the clinical image of subject's heart relative to the at least one clinical image from the reference sample is indicative of progression of cardiovascular disease in the subject.

In various embodiments, methods are provided for identifying at least one biomarker of cardiovascular disease in a subject, comprising: obtaining at least one clinical image of a heart of the subject; detecting and/or measuring and/or quantifying at least one iron deposit in the heart of the subject from the at least one clinical image of the heart of the subject; correlating the at least one iron deposit in the at least one clinical image of the heart of the subject to at least one microbleed in the heart of the subject so as to obtain a biomarker signature for the subject; and comparing the biomarker signature from the subject to a biomarker signature from a reference sample, wherein the at least one biomarker of cardiovascular disease is identified.

In various embodiments, methods are provided for identifying and/or assessing cardiovascular disease in a subject, comprising: obtaining at least one clinical image of a heart of the subject; detecting and/or measuring and/or quantifying at least one iron deposit in the heart of the subject from the at least one clinical image; correlating the at least one iron deposit in the heart of the subject to at least one microbleed in the heart of the subject so as to obtain a biomarker signature for the subject; and comparing the biomarker signature from the subject to a biomarker signature from a reference sample, wherein a change in the biomarker signature from the subject relative to the biomarker signature from the reference sample is indicative of cardiovascular disease in the subject.

In various embodiments, methods are provided for detecting cardiovascular disease in a subject, comprising: obtaining at least one clinical image of a heart of the subject; detecting and/or measuring and/or quantifying at least one iron deposit in the heart of the subject from the at least one clinical image; correlating the at least one iron deposit in the heart of the subject to at least one microbleed in the heart of the subject so as to obtain a biomarker signature for the subject; and comparing the biomarker signature from the subject to a biomarker signature from a reference sample, wherein a change in the biomarker signature from the subject relative to the biomarker signature from the reference sample is indicative of and/or a detection of cardiovascular disease in the subject.

In various embodiments, methods are provided for prognosing cardiovascular disease in a subject, comprising: obtaining at least one clinical image of a heart of the subject; detecting and/or measuring and/or quantifying at least one iron deposit in the heart of the subject from the at least one clinical image; correlating the at least one iron deposit in the heart of the subject to at least one microbleed in the heart of the subject so as to obtain a biomarker signature for the subject; and comparing the biomarker signature from the subject to a biomarker signature from a reference sample, wherein a change in the biomarker signature from the subject relative to the biomarker signature from the reference sample is a prognosis of cardiovascular disease in the subject.

In various embodiments, methods are provided for diagnosing cardiovascular disease in a subject, comprising: obtaining at least one clinical image of a heart of the subject; detecting and/or measuring and/or quantifying at least one iron deposit in the heart of the subject from the at least one clinical image; correlating the at least one iron deposit in the heart of the subject to at least one microbleed in the heart of the subject so as to obtain a biomarker signature for the subject; and comparing the biomarker signature from the subject to a biomarker signature from a reference sample, wherein a change in the biomarker signature from the subject relative to the biomarker signature from the reference sample is a diagnosis of cardiovascular disease in the subject.

In various embodiments, methods are provided for determining progression cardiovascular disease in a subject, comprising: obtaining at least one clinical image of a heart of the subject; detecting and/or measuring and/or quantifying at least one iron deposit in the heart of the subject from the at least one clinical image; correlating the at least one iron deposit in the heart of the subject to at least one microbleed in the heart of the subject so as to obtain a biomarker signature for the subject; and comparing the biomarker signature from the subject to a biomarker signature from a reference sample, wherein a change in the biomarker signature from the subject relative to the biomarker signature from the reference sample is indicative of progression of cardiovascular disease in the subject.

In some embodiments, methods are provided for assessing and/or determining the risk of developing cardiovascular disease in a subject, comprising: obtaining at least one clinical image of a heart of the subject; detecting and/or measuring and/or quantifying at least one iron deposit in the heart of the subject from the at least one clinical image; correlating the at least one iron deposit in the heart of the subject to at least one microbleed in the heart of the subject so as to obtain a biomarker signature for the subject; and comparing the biomarker signature from the subject to a biomarker signature from a reference sample, wherein a change in the biomarker signature from the subject relative to the biomarker signature from the reference sample is indicative of an increased risk of the subject developing cardiovascular disease.

In various embodiments, methods are provided for assessing and/or determining the risk of at least one cardiac arrhythmia in a subject, comprising: obtaining at least one clinical image of the heart of the subject; and detecting the presence of at least one iron deposit in the heart of the subject from the at least one clinical image, wherein the presence of the at least one iron deposit in the heart of the subject is indicative of the at least one microbleed in the heart of the subject, wherein at least one microbleed in the heart of the subject is indicative of an increased risk of at least one cardiac arrhythmia in the subject.

In various embodiments, methods are provided for assessing and/or determining the risk of sudden cardiac death in a subject, comprising: obtaining at least one clinical image of the heart of the subject; and detecting the presence of at least one iron deposit in the heart of the subject from the at least one clinical image, wherein the presence of the at least one iron deposit in the heart of the subject is indicative of the at least one microbleed in the heart of the subject, wherein at least one microbleed in the heart of the subject is indicative of an increased risk of sudden cardiac death in the subject.

In various embodiments, methods are provided for assessing and/or determining the risk of cardiovascular disease in a subject, comprising: obtaining at least one clinical image of the heart of the subject; and detecting the presence of at least one iron deposit in the heart of the subject from the at least one clinical image, wherein the presence of the at least one iron deposit in the heart of the subject is indicative of the at least one microbleed in the heart of the subject, wherein at least one microbleed in the heart of the subject is indicative of an increased risk of cardiovascular disease in a subject.

Various embodiments provide a method of identifying, detecting, prognosing, and/or diagnosing at least one microbleed in a heart of a subject, comprising detecting and/or measuring the presence of at least one iron deposit in the heart of the subject by performing a single-photon emission computed tomography (SPECT) scan of the heart, wherein the presence of the iron deposit in the heart of the subject is indicative of the at least one microbleed in the heart of the subject, and wherein the subject has been diagnosed with hypertension, hypertensive crisis, acute hypertensive crisis, prolonged hypertension, or a combination thereof. In some embodiments, a method of identifying a myocardial microbleed in a subject comprising performing SPECT scan of the heart or of the myocardium of the subject to detect the presence of iron deposit in the heart or the myocardium, respectively, wherein the presence indicates at least one myocardial microbleed. In some embodiments, a method of detecting a myocardial microbleed in a subject comprising performing SPECT scan of the heart or of the myocardium of the subj ect to detect the presence of iron deposit in the heart or the myocardium, respectively, wherein the presence indicates at least one myocardial microbleed. In some embodiments, a method of prognosing a myocardial microbleed in a subject comprising performing SPECT scan of the heart or of the myocardium of the subject to detect the presence of iron deposit in the heart or the myocardium, respectively, wherein the presence indicates at least one myocardial microbleed. In some embodiments, a method of diagnosing a myocardial microbleed in a subject comprising performing SPECT scan of the heart or of the myocardium of the subj ect to detect the presence of iron deposit in the heart or the myocardium, respectively, wherein the presence indicates at least one myocardial microbleed.

In some embodiments, a SPECT scan is replaced with, or performed in addition to, measuring the level of a hemoglobin breakdown product or a level of a ferroptosis marker in the blood of the subject, wherein the presence of the product or marker is indicative of at least one myocardial microbleed. In some embodiments, this is for a method of identifying a microbleed in the heart or the myocardium of the subject. In some embodiments, this is for a method of detecting a microbleed in the heart or the myocardium of the subject. In some embodiments, this is for a method of prognosing a microbleed in the heart or the myocardium of the subject. In some embodiments, this is for a method of diagnosing a microbleed in the heart or the myocardium of the subject.

Various embodiments provide a method of identifying, detecting, prognosing, and/or diagnosing at least one microbleed in the myocardium of a subject, comprising detecting and/or measuring the presence of at least one iron deposit in the heart of the subject by performing a single-photon emission computed tomography (SPECT) scan of the myocardium of the subject, wherein the presence of the iron deposit in the heart of the subject is indicative of the at least one microbleed in the myocardium or the heart of the subject, and wherein the subject has been diagnosed with hypertension, hypertensive crisis, acute hypertensive crisis, prolonged hypertension, or a combination thereof.

Various embodiments provide a method of identifying, detecting, prognosing, and/or diagnosing at least one microbleed in a heart of a subject, comprising detecting and/or measuring the presence of at least one iron deposit in the heart of the subject by performing a positron emission tomography (PET) scan of the heart, wherein the presence of the iron deposit in the heart of the subject is indicative of the at least one microbleed in the heart of the subject, and wherein the subject has been diagnosed with hypertension, hypertensive crisis, acute hypertensive crisis, prolonged hypertension, or a combination thereof. In some embodiments, a method of identifying a cardiac or myocardial microbleed in a subject comprising performing PET scan of the heart or of the myocardium of the subject to detect the presence of iron deposit in the heart or the myocardium, respectively, wherein the presence indicates at least one cardiac or myocardial microbleed. In some embodiments, a method of detecting a cardiac or myocardial microbleed in a subject comprising performing PET scan of the heart or of the myocardium of the subj ect to detect the presence of iron deposit in the heart or the myocardium, respectively, wherein the presence indicates at least one myocardial microbleed. In some embodiments, a method of prognosing a cardiac or myocardial microbleed in a subject comprising performing PET scan of the heart or of the myocardium of the subject to detect the presence of iron deposit in the heart or the myocardium, respectively, wherein the presence indicates at least one cardiac or myocardial microbleed. In some embodiments, a method of diagnosing a cardiac or myocardial microbleed in a subject comprising performing PET scan of the heart or of the myocardium of the subject to detect the presence of iron deposit in the heart or the myocardium, respectively, wherein the presence indicates at least one cardiac or myocardial microbleed.

Various embodiments provide a method of identifying, detecting, prognosing, and/or diagnosing at least one microbleed in a heart of a subject, comprising detecting and/or measuring the presence of at least one iron deposit in the heart of the subject by performing a computer tomography (CT) scan of the heart, wherein the presence of the iron deposit in the heart of the subject is indicative of the at least one microbleed in the heart of the subject, and wherein the subject has been diagnosed with hypertension, hypertensive crisis, acute hypertensive crisis, prolonged hypertension, or a combination thereof. In some embodiments, a method of identifying a cardiac or myocardial microbleed in a subject comprising performing CT scan of the heart or of the myocardium of the subject to detect the presence of iron deposit in the heart or the myocardium, respectively, wherein the presence indicates at least one cardiac or myocardial microbleed. In some embodiments, a method of detecting a cardiac or myocardial microbleed in a subject comprising performing CT scan of the heart or of the myocardium of the subj ect to detect the presence of iron deposit in the heart or the myocardium, respectively, wherein the presence indicates at least one myocardial microbleed. In some embodiments, a method of prognosing a cardiac or myocardial microbleed in a subject comprising performing CT scan of the heart or of the myocardium of the subject to detect the presence of iron deposit in the heart or the myocardium, respectively, wherein the presence indicates at least one cardiac or myocardial microbleed. In some embodiments, a method of diagnosing a cardiac or myocardial microbleed in a subject comprising performing CT scan of the heart or of the myocardium of the subject to detect the presence of iron deposit in the heart or the myocardium, respectively, wherein the presence indicates at least one cardiac or myocardial microbleed.

Various embodiments provide a method of identifying, detecting, prognosing, and/or diagnosing at least one microbleed in a heart of a subject, comprising detecting and/or measuring the presence of at least one iron deposit in the heart of the subject by performing a ultrasound (US) scan of the heart, wherein the presence of the iron deposit in the heart of the subject is indicative of the at least one microbleed in the heart of the subject, and wherein the subject has been diagnosed with hypertension, hypertensive crisis, acute hypertensive crisis, prolonged hypertension, or a combination thereof. In some embodiments, a method of identifying a cardiac or myocardial microbleed in a subject comprising performing US scan of the heart or of the myocardium of the subject to detect the presence of iron deposit in the heart or the myocardium, respectively, wherein the presence indicates at least one cardiac or myocardial microbleed. In some embodiments, a method of detecting a cardiac or myocardial microbleed in a subject comprising performing US scan of the heart or of the myocardium of the subject to detect the presence of iron deposit in the heart or the myocardium, respectively, wherein the presence indicates at least one myocardial microbleed. In some embodiments, a method of prognosing a cardiac or myocardial microbleed in a subject comprising performing US scan of the heart or of the myocardium of the subject to detect the presence of iron deposit in the heart or the myocardium, respectively, wherein the presence indicates at least one cardiac or myocardial microbleed. In some embodiments, a method of diagnosing a cardiac or myocardial microbleed in a subject comprising performing US scan of the heart or of the myocardium of the subj ect to detect the presence of iron deposit in the heart or the myocardium, respectively, wherein the presence indicates at least one cardiac or myocardial microbleed.

Various embodiments provide a method of identifying, detecting, prognosing, and/or diagnosing at least one microbleed in a heart of a subject, comprising detecting and/or measuring the presence of at least one iron deposit in the heart of the subject by performing a magnetic resonance imaging (MRI) scan of the heart, wherein the presence of the iron deposit in the heart of the subject is indicative of the at least one microbleed in the heart of the subject, and wherein the subject has been diagnosed with hypertension, hypertensive crisis, acute hypertensive crisis, prolonged hypertension, or a combination thereof. In some embodiments, a method of identifying a cardiac or myocardial microbleed in a subject comprising performing MRI scan of the heart or of the myocardium of the subject to detect the presence of iron deposit in the heart or the myocardium, respectively, wherein the presence indicates at least one cardiac or myocardial microbleed. In some embodiments, a method of detecting a cardiac or myocardial microbleed in a subject comprising performing MRI scan of the heart or of the myocardium of the subj ect to detect the presence of iron deposit in the heart or the myocardium, respectively, wherein the presence indicates at least one myocardial microbleed. In some embodiments, a method of prognosing a cardiac or myocardial microbleed in a subject comprising performing MRI scan of the heart or of the myocardium of the subject to detect the presence of iron deposit in the heart or the myocardium, respectively, wherein the presence indicates at least one cardiac or myocardial microbleed. In some embodiments, a method of diagnosing a cardiac or myocardial microbleed in a subject comprising performing MRI scan of the heart or of the myocardium of the subject to detect the presence of iron deposit in the heart or the myocardium, respectively, wherein the presence indicates at least one cardiac or myocardial microbleed.

Various embodiments provide a method of identifying, detecting, prognosing, and/or diagnosing at least one microbleed in a heart of a subject, comprising detecting and/or measuring the presence of at least one iron deposit in the heart of the subject measuring from blood of the subject a level of a hemoglobin breakdown product, wherein the presence of the iron deposit in the heart of the subject is indicative of the at least one microbleed in the heart of the subject, and wherein the subject has been diagnosed with hypertension, hypertensive crisis, acute hypertensive crisis, prolonged hypertension, or a combination thereof. Exemplary hemoglobin breakdown products include iron, bilirubin, globin, and a combination thereof
Various embodiments provide a method of identifying, detecting, prognosing, and/or diagnosing at least one microbleed in a heart of a subject, comprising detecting and/or measuring the presence of at least one iron deposit in the heart of the subject measuring from blood of the subject a level of a ferroptosis marker, wherein the presence of the iron deposit in the heart of the subject is indicative of the at least one microbleed in the heart of the subject, and wherein the subject has been diagnosed with hypertension, hypertensive crisis, acute hypertensive crisis, prolonged hypertension, or a combination thereof. Exemplary ferroptosis markers include those listed in relation to description of ferroptosis above. In some embodiments, the ferroptosis marker in the methods comprises glutathione peroxidase 4 (GPX4). A method is provided for identifying a cardiac or myocardium microbleed in a subject, comprising or consisting of measuring from blood of the subject a level of a ferroptosis marker comprising GPX4, wherein the presence or a higher level of GPX4 than a reference subject is indicative of a cardiac or myocardium microbleed of the subject. A method is provided for detecting a cardiac or myocardium microbleed in a subject, comprising or consisting of measuring from blood of the subject a level of a ferroptosis marker comprising GPX4, wherein the presence or a higher level of GPX4 than a reference subject is indicative of a cardiac or myocardium microbleed of the subject. A method is provided for prognosing a cardiac or myocardium microbleed in a subject, comprising or consisting of measuring from blood of the subject a level of a ferroptosis marker comprising GPX4, wherein the presence or a higher level of GPX4 than a reference subject is indicative of a cardiac or myocardium microbleed of the subject. A method is provided for diagnosing a cardiac or myocardium microbleed in a subject, comprising or consisting of measuring from blood of the subject a level of a ferroptosis marker comprising GPX4, wherein the presence or a higher level of GPX4 than a reference subject is indicative of a cardiac or myocardium microbleed of the subject. In further embodiments, the ferroptosis markers in the methods further include another 1, 2, 3, 4, 5, 6, 7, 8, 9, or 10 markers in addition to GPX4.

In various embodiments, techniques of using MRI to detect iron, especially for the heart tissue, include using MRI sequences selected from T2*, T1, T2, proton density-weighted sequence, and susceptibility-weighted imaging (SWI), thereby obtaining T1 maps, T1-weighted images, susceptibility-weighted images including quantitative susceptibility maps, T2 map, T2* map, T2 or T2* map, T2 or T2*-weighted, corrected T2 or T2*-weighted images. Other techniques to detect iron in the heart tissue include some invasive techniques, such as histological staining or inductively coupled plasma-mass spectrometry on heart tissue specimens. Details of detecting iron or iron-deposits in the heart tissue are described in U.S. Pat. No. 10,694,961, q.v..

In some embodiments, the subject is human. In some embodiments, the subject has at least one selected from the group consisting of hypertension, hypertensive crisis, acute hypertensive crisis, prolonged hypertension, and a combination thereof.

In some embodiments, the subject does not have myocardial infarction or reperfusion therapy-induced hemorrhage; in some further aspects, the subject does not have hemorrhagic myocardial infarction; in some further aspects, the subject has not undergone reperfusion therapy, and therefore would not have reperfusion hemorrhage. In some embodiments, the subject has not had a myocardial infarction or a reperfusion therapy in the past 3 days, 7 days, 1 month, 2 months, 3 months, 4 months, 5 months, 6 months, 7 months, 8 months, 9 months, 10 months, 11 months, 12 months, 2 years, 3 years, or 5 years. In some embodiments, a subject in need of one or more methods disclosed herein is a subject with any one or more characteristics above; and in some embodiments, a subject in need thereof desires to identify, detect, prognose or diagnose myocardial microbleeds.

In some embodiments, the subject has idiopathic hypertension. In some embodiments, the subject has long-standing or chronic idiopathic hypertension who develops hypertensive microbleeds. In some embodiments, the subject has symptoms, for example, transient focal symptoms associated with an acute microbleed, such as a sudden tingling sensation (e.g., in a side or both sides of face, arms, chest, limbs), heaviness of the arm and/or legs, double vision and/or nausea. In some embodiments, the subject has been diagnosed with hypertension. In some embodiments, the subject has been diagnosed with hypertension and has been taken anticoagulant medications (e.g., apixaban, dabigatran, edoxaban, enoxaparin, heparin, rivaroxaban, warfarin). In some embodiments, the subject has been diagnosed with stress-induced conditions (cardiomyopathy), such as takotsubo cardiomyopathy.

In some embodiments, methods of treating a subject with myocardial microbleed are provided. In some embodiments, one or more of the methods disclosed above for identifying, detecting, prognosing or diagnosing a myocardial microbleed, or thereby an associated cardiovascular disease, further comprise treating the subject with a treatment and/or selecting a treatment for the subject and/or providing a treatment to the subject and/or administering a treatment to the subject. In some embodiments, the treatment is a therapeutic composition including at least one chelating agent. In some embodiments, the chelating agent is selected from the group consisting of Deferoxamine (also known as desferrioxamine), Deferasirox, Deferiprone, hinokitiol, dexrazoxane, 2,2'-bipyridyl, pyridoxal isonicotinoyl hydrazone, salicylaldehyde isonicotinoyl hydrazone, exochelins (including desferri-exochelins), and combinations thereof. Desferri-exochelins are hexadentate molecules, and by forming a one-to-one binding relationship with iron, they prevent free radical reactions; whereas deferiprone is a bidentate molecule and desferasirox is a tridentate molecule. In some embodiments, the chelating agent is an iron chelator. In some embodiments, the iron chelator is selected from the group consisting of an intracellular iron chelator, extracellular iron chelator, or combination thereof. In some embodiments, the intracellular iron chelator chelates ferrous iron, ferric iron, and combinations thereof. In some embodiments, the extracellular iron chelator chelates ferrous iron, ferric iron, and combinations thereof. In some embodiments, the treatment is selected from the group consisting of at least one heme-binding protein, at least one heme-degrading protein, and combinations thereof. In some embodiments, the method further comprises administering to the subject an amount of a factor effective to increase an amount of at least one selected from the group consisting of at least one heme-binding protein, at least one heme-degrading protein, at least one heme-blocking protein, and combinations thereof in the subject. In some embodiments, the heme-binding protein is selected from the group consisting of hemopexin, haptoglobin, albumin, ferritin, alpha1-microglobulin, alpha1-antitrypsin, glutathione-S-transferases, heme binding protein / Liver fatty acid binding protein, heme-binding protein 23 / peroxiredoxin, p22 heme binding protein and glyceraldehyde-3-phosphate dehydrogenase, and combinations thereof. In some embodiments, the heme-degrading protein is selected from the group consisting of heme oxygenase 1, hinokitiol, nuclear factor E2 related factor 2 (Nrf2), and combinations thereof. In some embodiments, heme-blocking proteins refer to those that bind to a protein that would otherwise transport heme, thus rendering the bound protein ineffective in its function so as to reduce uptake of heme into the cell; therefore, an indirect blocking of heme. For example, a heme-blocking agent can be an inhibitor of heme carrier protein 1 (HCP1), or an inhibitor of divalent metal transporter 1 (DMT1). In another example, a heme-blocking agent is hepcidin, which binds to ferroportin and the complex undergoes endocytosis and degradation, resulting in the loss of ferroportin from the cellular surface and therefore induces a secondary decrease in the release of iron from the cell. In some embodiments, the factor is selected from FLVCR1a (Feline leukemia virus subgroup C receptor 1a), ABCG2 (ATP-binding cassette subfamily G member 2), FLVCR2, and combinations thereof. In some embodiments, the heme binding protein is a heme scavenger.

Additional agents can be included or administered in combination with the therapeutic composition, including but are not limited to an anti-inflammatory agent, a lipid-lowering agent, an agent capable of promoting clearance of iron with enhanced macrophage activity, a phagocytosis-enhancing agent, or an agent capable of disrupting the biosynthesis of iron oxide crystals or preventing aggregation of nanocrystals, or a combination thereof. When administered "in combination," agents can be premixed before administration, administered simultaneously, or administered sequentially (including continuously or separated by a no-administration period). Exemplary anti-inflammatory agents include colchicine, a corticosteroid, nonsteroidal anti-inflammatory drug (NSAID), anti-IL-1beta (e.g., Anakinra), anti-TNF-a (e.g., Etanercept and Infliximab), anti-IL-6 (e.g., Tocilizumab), anti-MMP (e.g., PG-116800 and Doxycycline), macrophage modulators (e.g., phosphatidylserine-presenting liposomes), NLRP3 inflammasome inhibitors (e.g., 16673-34-0 (5-chloro-2-methoxy-N-[2-(4-sulfamoylphenyl)ethyl]benzamide)), inflammasome antagonists (e.g., P2X7 antagonist), or anti-diabetic medications (for example, insulin (e.g., Humulin, Novolin, Humalog), metformin (e.g., Glucophage, Glucophage XR, Fortamet, Glumetza, Riomet), sulfonylureas, meglitinides, incretin mimetics, biguanides, amylinomimetic agent (e.g., Pramlintide), lipase inhibitors such as orlistat (e.g., Xenical, Alli), thiazolidinediones, Pioglitazone (e.g., Actos), Rosiglitazone (e.g., Avandia), corticosteroids such as Prednisone (e.g., Rayos), dipeptidyl peptidase-4 inhibitors, SGLT2 inhibitors, and glucagon-like peptide-1 analogs or agonists such as Exenatide (e.g., Bydureon, Byetta) and Liraglutide (e.g., Victoza)). Exemplary lipid-lowering agents include statin, cholesterol absorption inhibitors (e.g., ezetimbie), bile-acid-binding resins/sequestrants (e.g., Cholestyramine), niacin, or vitamin B3.

In some embodiments, the chelating agent, the heme-binding protein, the heme-blocking protein, and/or the heme-degrading protein is administered for a period of time from 1 day to 1 year, e.g., 1 week, 1 month, 2 months, 3 months, 4 months, 5 months, 6 months, 7 months, 8 months, 9 months, 10 months, 11 months or 1 year, or at least for 1 year, 2 years, 3 years, 4 years, or 5 years.

In some embodiments, the therapeutic composition or the treatment is initiated or administered to the subject immediately following the symptoms appear, or within 5 minutes, 10 minutes, 30 minutes, 1 hour, 2 hours, 4 hours, 12 hours, 24 hours, 2 days, 3 days, 4 days, 5 days, 6 days, 1 week, 2 weeks, 3 weeks, or a month from when the symptoms appear. In further embodiments, the therapeutic composition or the treatment is initiated or administered to the subject after the subject is diagnosed to confirm presence of iron deposits or microbleeding in the myocardium.

Further embodiments provide that the treatment methods also include monitoring or reassessing the subject. In some embodiments, the methods include imaging the subject's heart after an administration (a dose) of the composition to detect a level of iron deposit in the myocardium or another portion of the heart. In further embodiments, the methods include administering to the subject a subsequent dose of the composition if a same or higher level of iron deposit is indicated in the myocardium or the other portion of the heart relative to the level before a previous dose was administered to the subject, or discontinuing administration of the composition if iron deposit is absent in the myocardium or the other portion of the heart. In a further embodiment, iron levels at or above 1-5 µg/g, 5-10 µg/g, 0.01 mg/g, 0.02 mg/g, 0.03 mg/g, 0.04 mg/g of tissue in the myocardium is indicative of occurred or reoccurring myocardial microbleeding in the subject.

Exemplary dosages of a chelator, a heme-binding protein, a heme-degrading protein, a heme-blocking protein, or a factor disclosed above, per unit weight of a subject in the methods above include 10-100 µg, 100-200 µg, 200-300 µg, 300-400 µg, 400-500 µg, 500-600 µg, 600-700 µg, 700-800 µg, 800-900 µg, 1-5 mg, 5-10 mg, 10-20 mg, 20-30 mg, 30-40 mg, 40-50 mg, 50-60 mg, 60-70 mg, 70-80 mg, 80-90 mg, 90-100 mg, 100-200 mg, 200-300 mg, 300-400 mg, 400 mg-500 mg, 500 mg-1g, or 1g-10g. Unit weight of a subject can be per kg of body weight or per subject. In another embodiment, the chelating agents (for example Deferoxamine, Deferasirox, Deferiprone) may be used at a dose of any one or more of 100-200 mg/period, 200-300mg/period, 300-400mg/period, 400-500mg/period, 500-600mg/period, 600-700mg/period, 700-800mg/period, 800-900mg/period, 900-100mg/period or a combination thereof. A period of dosing can be a day, within 24 hours, 48 hours, 96 hours, a week, every two weeks, or a month.

In some embodiments, the iron deposit comprises iron oxide. In some embodiments, the iron deposit comprises at least one compound comprising iron. In some embodiments, the compound is selected from the group consisting of heme, heme derivative, and combinations thereof. In some embodiments, the iron is in any oxidation state possible. In some embodiments, the oxidation state is selected from +2, +2/+3, +3, +4, and combinations thereof. In some embodiments, the iron is selected from the group consisting of ferrous iron, ferric iron, and combinations thereof. In some embodiments, the iron is in any form.

In some embodiments, the portion of the heart is selected from the group consisting of epicardium, myocardium, endocardium, valvular tissue, and a combination thereof. In some embodiments, the portion of the heart is selected from the group consisting of valvular tissue, endocardium, pulmonary valve, brachiocephalic artery, superior vena cava, right pulmonary artery, right pulmonary veins, tricuspid valve, right ventricle, inferior vena cava, descending aorta, papillary muscle, interventricular septum, epicardium, left ventricle, myocardium, mitral valve, aortic valve, left pulmonary veins, left pulmonary artery, aorta, left subclavian artery, pulmonary trunk, left common carotid artery, and a combination thereof.

In some embodiments, the change is in the at least one iron deposit. In some embodiments, the change is in at least one selected from the group consisting of an amount, quantity, concentration, shape, size, and pattern of the at least one iron deposit.

In some embodiments, the reference sample is a heart, heart tissue, or portion of a heart from a control subject, wherein the control subject does not have at least one iron deposit in the heart, heart tissue, or portion of the heart. In some embodiments, the reference sample is a heart, heart tissue, or portion of a heart from a control subject, wherein the control subject does not have at least one microbleed in the heart, heart tissue, or portion of a heart. In some embodiments, the reference sample is the heart, heart tissue, or portion of a heart from the subject at an earlier point in time.

In some embodiments, the cardiovascular disease is selected from the group consisting of infarcted myocardium, coronary artery disease, coronary heart disease, ischemic heart disease, cardiomyopathy, stroke, hypertensive heart disease, heart failure, pulmonary heart disease, ischemic syndrome, coronary microvascular disease, cardiac dysrhythmias, rheumatic heart disease, aortic aneurysms, cardiomyopathy, atrial fibrillation, congenital heart disease, endocarditis, inflammatory heart disease, endocarditis, inflammatory cardiomegaly, myocarditis, valvular heart disease, cerebrovascular disease, peripheral artery disease, acute stress-induced cardiomyopathy (Takotsubo cardiomyopathy), diffuse myocardial fibrosis, and a combination thereof.

In some embodiments, the reference sample is a heart, heart tissue, or portion of a heart from the subject before the subject is treated for the cardiovascular disease. In some embodiments, the reference sample is a heart, heart tissue, or portion of a heart from a subject that has been successfully treated for the cardiovascular disease.

In some embodiments, the reference sample is a heart, heart tissue, or portion of a heart from a control subject, wherein the control subject does not have the cardiovascular disease. In some embodiments, the reference sample is a heart, heart tissue, or portion of a heart from a control subject, wherein the control subject has the cardiovascular disease.

In some embodiments, the reference sample is from a heart, heart tissue, or portion of a heart of a subject that has been successfully treated for the cardiovascular disease. In some embodiments, the reference sample is from the heart, heart tissue, or portion of a heart of the subject at an earlier point in time.

In some embodiments, obtaining at least one magnetic resonance image comprises operating a magnetic resonance imaging machine/scanner to obtain the at least one magnetic resonance image.

In some embodiments, the heart tissue is selected from the group consisting of valvular tissue, endocardium, pulmonary valve, brachiocephalic artery, superior vena cava, right pulmonary artery, right pulmonary veins, tricuspid valve, right ventricle, inferior vena cava, descending aorta, papillary muscle, interventricular septum, epicardium, left ventricle, myocardium, mitral valve, aortic valve, left pulmonary veins, left pulmonary artery, aorta, left subclavian artery, pulmonary trunk, left common carotid artery, and a combination thereof.

### EXAMPLES

The invention will be further explained by the following Examples, which are intended to be purely exemplary of the invention, and should not be considered as limiting the invention in any way. The following examples are provided to better illustrate the claimed invention and are not to be interpreted as limiting the scope of the invention. To the extent that specific materials are mentioned, it is merely for purposes of illustration and is not intended to limit the invention. One skilled in the art may develop equivalent means or reactants without the exercise of inventive capacity and without departing from the scope of the invention.

### Example 1

FIG. 1A shows iron in the myocardial tissue and FIG. 1B shows the ensuing myocardial fibrosis in wild-type rats treated with angiotensin II to induce hypertension.

To provide aspects of the present disclosure, embodiments may employ any number of programmable processing devices that execute software or stored instructions. Physical processors and/or machines employed by embodiments of the present disclosure for any processing or evaluation may include one or more networked (Internet, cloud, WAN, LAN, satellite, wired or wireless (RF, cellular, WiFi, Bluetooth, etc.)) or non-networked general purpose computer systems, microprocessors, filed programmable gate arrays (FPGAs), digital signal processors (DSPs), micro-controllers, smart devices (e.g., smart phones), computer tablets, handheld computers, and the like, programmed according to the teachings of the exemplary embodiments. In addition, the devices and subsystems of the exemplary embodiments can be implemented by the preparation of application-specific integrated circuits (ASICs) or by interconnecting an appropriate network of conventional component circuits. Thus, the exemplary embodiments are not limited to any specific combination of hardware circuitry and/or software.

Stored on any one or on a combination of computer readable media, the exemplary embodiments of the present disclosure may include software for controlling the devices and subsystems of the exemplary embodiments, for driving the devices and subsystems of the exemplary embodiments, for enabling the devices and subsystems of the exemplary embodiments to interact with a human user, and the like. Such software can include, but is not limited to, device drivers, firmware, operating systems, development tools, applications software, database management software, and the like. Computer code devices of the exemplary embodiments can include any suitable interpretable or executable code mechanism, including but not limited to scripts, interpretable programs, dynamic link libraries (DLLs), Java classes and applets, complete executable programs, and the like. Moreover, processing capabilities may be distributed across multiple processors for better performance, reliability, cost, or other benefits.

Common forms of computer-readable media may include, for example, a floppy disk, a flexible disk, a hard disk, magnetic tape, any other suitable magnetic medium, a CD-ROM, CDRW, DVD, any other suitable optical medium, punch cards, paper tape, optical mark sheets, any other suitable physical medium with patterns of holes or other optically recognizable indicia, a RAM, a PROM, an EPROM, a FLASH-EPROM, any other suitable memory chip or cartridge, a carrier wave or any other suitable medium from which a computer can read. Such storage media can also be employed to store other types of data, e.g., data organized in a database, for access, processing, and communication by the processing devices.

The various methods and techniques described above provide a number of ways to carry out the application. Of course, it is to be understood that not necessarily all objectives or advantages described can be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as taught or suggested herein. A variety of alternatives are mentioned herein. It is to be understood that some embodiments specifically include one, another, or several features, while others specifically exclude one, another, or several features, while still others mitigate a particular feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be employed in various combinations by one of ordinary skill in this art to perform methods in accordance with the principles described herein. Among the various elements, features, and steps some will be specifically included and others specifically excluded in diverse embodiments.

Although the application has been disclosed in the context of certain embodiments and examples, it will be understood by those skilled in the art that the embodiments of the application extend beyond the specifically disclosed embodiments to other alternative embodiments and/or uses and modifications and equivalents thereof.

Various embodiments of this application are described herein, including the best mode known to the inventors for carrying out the application. Variations on those embodiments will become apparent to those of ordinary skill in the art upon reading the foregoing description. It is contemplated that skilled artisans can employ such variations as appropriate, and the application can be practiced otherwise than specifically described herein. Accordingly, many embodiments of this application include all modifications and equivalents of the subject matter recited in the claims appended hereto as permitted by applicable law. Moreover, any combination of the above-described elements in all possible variations thereof is encompassed by the application unless otherwise indicated herein or otherwise clearly contradicted by context.

It is to be understood that the embodiments of the application disclosed herein are illustrative of the principles of the embodiments of the application. Other modifications that can be employed can be within the scope of the application. Thus, by way of example, but not of limitation, alternative configurations of the embodiments of the application can be utilized in accordance with the teachings herein. Accordingly, embodiments of the present application are not limited to that precisely as shown and described.

Various embodiments of the invention are described above in the Detailed Description. While these descriptions directly describe the above embodiments, it is understood that those skilled in the art may conceive modifications and/or variations to the specific embodiments shown and described herein. Any such modifications or variations that fall within the purview of this description are intended to be included therein as well. Unless specifically noted, it is the intention of the inventors that the words and phrases in the specification and claims be given the ordinary and accustomed meanings to those of ordinary skill in the applicable art(s).

The foregoing description of various embodiments of the invention known to the applicant at this time of filing the application has been presented and is intended for the purposes of illustration and description. The present description is not intended to be exhaustive nor limit the invention to the precise form disclosed and many modifications and variations are possible in the light of the above teachings. The embodiments described serve to explain the principles of the invention and its practical application and to enable others skilled in the art to utilize the invention in various embodiments and with various modifications as are suited to the particular use contemplated. Therefore, it is intended that the invention not be limited to the particular embodiments disclosed for carrying out the invention.

While particular embodiments of the present invention have been shown and described, it will be obvious to those skilled in the art that, based upon the teachings herein, changes and modifications may be made without departing from this invention and its broader aspects and, therefore, the appended claims are to encompass within their scope all such changes and modifications as are within the scope of this invention.

## Claims

1. A method of identifying, detecting, prognosing, and/or diagnosing at least one microbleed in a heart of a subject in need thereof, comprising:
detecting and/or measuring the presence of at least one iron deposit in the heart of the subject by measuring in blood taken from the subject a level of a hemoglobin breakdown product or a level of a ferroptosis marker,
wherein the presence of the iron deposit in the heart of the subject is indicative of the at least one microbleed in the heart of the subject, and
wherein the subject has been diagnosed with hypertension, hypertensive crisis, acute hypertensive crisis, prolonged hypertension, or a combination thereof.

2. The method of claim 1, wherein the subject has been diagnosed with hypertension.

3. The method of claim 1, wherein detecting and/or measuring the presence of the at least one iron deposit includes measuring in blood taken from the subject a level of a hemoglobin breakdown product or a level of a ferroptosis marker, wherein the hemoglobin breakdown product is selected from the group consisting of iron, bilirubin, globin, and a combination thereof, and the ferroptosis markers comprises glutathione peroxidase 4 (GPX4).

4. The method of claim 1, further comprising:
comparing the level of the hemoglobin breakdown product or the level of the ferroptosis marker of the subject to that obtained from a reference sample.

5. The method of claim 4, wherein the presence of at least one iron deposit in the heart of the subject relative to the reference sample is indicative of, or a prognosis or a diagnosis of, at least one microbleed in the heart of the subject.

6. The method of claim 4 for determining progression of the at least one microbleed in the heart of the subject, wherein a change in the level of the hemoglobin breakdown product or the level of the ferroptosis marker of the subject relative to that obtained from the reference sample is indicative of progression of at least one microbleed in the heart of the subject.

7. A method of identifying, detecting, prognosing, diagnosing, and/or determining progression of a cardiovascular disease in a subject in need thereof, comprising:
detecting at least one iron deposit in the heart of the subject by measuring in blood taken from the subject a level of a hemoglobin breakdown product or a level of a ferroptosis marker, wherein detection of the iron deposit in the heart of the subject is indicative of a presence of at least one microbleed in the heart of the subject; and
comparing the level of the hemoglobin breakdown product or the level of the ferroptosis marker of the subject to that obtained from a reference sample, wherein a change in the level of the hemoglobin breakdown product or in the level of the ferroptosis marker of the subject relative to that obtained from the reference sample is indicative of, or a prognosis or a diagnosis of, a cardiovascular disease, or indicative of progression of the cardiovascular disease, in the subject,
wherein the subject has been diagnosed with hypertension, hypertensive crisis, acute hypertensive crisis, prolonged hypertension, or a combination thereof.

8. The method of claim 7, further comprising:
correlating the at least one iron deposit indicated by the level of the hemoglobin breakdown product or by the level of the ferroptosis marker, of the subject to at least one microbleed in the heart of the subject so as to obtain a biomarker signature from the subject; and
wherein the comparing step compares the biomarker signature obtained from the subject to a biomarker signature from a reference sample, thereby identifying at least one biomarker of the cardiovascular disease, and
wherein a change in the biomarker signature from the subject relative to the biomarker signature from the reference sample is indicative of, or a detection, a prognosis, or a diagnosis of, the cardiovascular disease, or indicative of progression of the cardiovascular disease, in the subject.

9. The method of claim 7, wherein the cardiovascular disease comprises cardiomyopathy, myocardial fibrosis, cardiac arrhythmia, or sudden cardiac death.

10. The method of any of claims 1-6, wherein the method is for:
a) identifying at least one microbleed in the heart of the subject;
b) detecting at least one microbleed in the heart of the subject;
c) prognosing at least one microbleed in the heart of the subject; or
d) diagnosing at least one microbleed in the heart of the subject.

11. The method of any of claims 7-9, wherein the method is for:
a) identifying the cardiovascular disease in the subject or identifying a subject with the cardiovascular disease;
b) detecting the cardiovascular disease in the subject;
c) prognosing the cardiovascular disease in the subject; or
d) determining progression of the cardiovascular disease in the subject.

12. The method of claim 1 or 7, wherein the subject does not have myocardial infarction or reperfusion therapy-induced hemorrhage, or wherein the subject has not had myocardial infarction or reperfusion therapy-induced hemorrhage in the past 3 days, 7 days, 1 month, 2 months, 3 months, 4 months, 5 months, 6 months, 7 months, 8 months, 9 months, 10 months, 11 months, 12 months, 2 years, 3 years, or 5 years.

## Patentansprüche

1. Verfahren zum Identifizieren, Nachweisen, Prognostizieren und/oder Diagnostizieren mindestens einer Mikroblutung in einem Herzen eines Subjekts, das dessen bedarf, umfassend:
Nachweisen und/oder Messen des Vorhandenseins mindestens einer Eisenablagerung im Herzen des Subjekts durch Messen eines Spiegels eines Hämoglobinabbauprodukts oder eines Spiegels eines Ferroptosemarkers in Blut, das dem Subjekt entnommen wurde,
wobei das Vorhandensein der Eisenablagerung im Herzen des Subjekts auf die mindestens eine Mikroblutung im Herzen des Subjekts hinweist und
wobei bei dem Subjekt Hypertonie, eine hypertensive Krise, eine akute hypertensive Krise, eine anhaltende Hypertonie oder eine Kombination davon diagnostiziert ist.

2. Verfahren nach Anspruch 1, wobei bei dem Subjekt Hypertonie diagnostiziert ist.

3. Verfahren nach Anspruch 1, wobei das Nachweisen und/oder Messen des Vorhandenseins der mindestens einen Eisenablagerung Messen eines Spiegels eines Hämoglobinabbauprodukts oder eines Spiegels eines Ferroptosemarkers in Blut, das dem Subjekt entnommen wurde, enthält, wobei das Hämoglobinabbauprodukt ausgewählt ist aus der Gruppe bestehend aus Eisen, Bilirubin, Globin und einer Kombination davon, und die Ferroptosemarker Glutathionperoxidase 4 (GPX4) umfasst.

4. Verfahren nach Anspruch 1, ferner umfassend:
Vergleichen des Spiegels des Hämoglobinabbauprodukts oder des Spiegels des Ferroptosemarkers des Subjekts mit demjenigen, der aus einer Referenzprobe erhalten wurde.

5. Verfahren nach Anspruch 4, wobei das Vorhandensein mindestens einer Eisenablagerung im Herzen des Subjekts relativ zu der Referenzprobe auf mindestens eine Mikroblutung im Herzen des Subjekts hinweist oder eine Prognose oder eine Diagnose einer solchen Mikroblutung darstellt.

6. Verfahren nach Anspruch 4 zum Bestimmen einer Progression der mindestens einen Mikroblutung im Herzen des Subjekts, wobei eine Änderung des Spiegels des Hämoglobinabbauprodukts oder des Spiegels des Ferroptosemarkers des Subjekts relativ zu demjenigen, der aus der Referenzprobe erhalten wurde, auf eine Progression mindestens einer Mikroblutung im Herzen des Subjekts hinweist.

7. Verfahren zum Identifizieren, Nachweisen, Prognostizieren, Diagnostizieren und/oder Bestimmen einer Progression einer kardiovaskulären Erkrankung bei einem Subjekt, das dessen bedarf, umfassend:
Nachweisen mindestens einer Eisenablagerung im Herzen des Subjekts durch Messen eines Spiegels eines Hämoglobinabbauprodukts oder eines Spiegels eines Ferroptosemarkers in Blut, das dem Subjekt entnommen wurde, wobei ein Nachweis der Eisenablagerung im Herzen des Subjekts auf ein Vorhandensein mindestens einer Mikroblutung im Herzen des Subjekts hinweist; und
Vergleichen des Spiegels des Hämoglobinabbauprodukts oder des Spiegels des Ferroptosemarkers des Subjekts mit demjenigen, der aus einer Referenzprobe erhalten wurde, wobei eine Änderung des Spiegels des Hämoglobinabbauprodukts oder des Spiegels des Ferroptosemarkers des Subjekts relativ zu demjenigen, der aus der Referenzprobe erhalten wurde, auf eine kardiovaskuläre Erkrankung hinweist oder eine Prognose oder eine Diagnose einer solchen Erkrankung darstellt oder auf eine Progression der kardiovaskulären Erkrankung in dem Subjekt hinweist,
wobei bei dem Subjekt Hypertonie, eine hypertensive Krise, eine akute hypertensive Krise, eine anhaltende Hypertonie oder eine Kombination davon diagnostiziert ist.

8. Verfahren nach Anspruch 7, ferner umfassend:
Korrelieren der mindestens einen Eisenablagerung, auf die durch den Spiegel des Hämoglobinabbauprodukts oder durch den Spiegel des Ferroptosemarkers hingewiesen wird, des Subjekts mit mindestens einer Mikroblutung im Herzen des Subjekts, um eine Biomarkersignatur von dem Subjekt zu erhalten; und
wobei der Schritt des Vergleichens die von dem Subjekt erhaltene Biomarkersignatur mit einer Biomarkersignatur von einer Referenzprobe vergleicht, wodurch mindestens ein Biomarker der kardiovaskulären Erkrankung identifiziert wird, und
wobei eine Änderung der Biomarkersignatur von dem Subjekt relativ zu der Biomarkersignatur von der Referenzprobe auf eine kardiovaskulären Erkrankung hinweist oder einen Nachweis, eine Prognose oder eine Diagnose derselben darstellt oder auf eine Progression der kardiovaskulären Erkrankung in dem Subjekt hinweist.

9. Verfahren nach Anspruch 7, wobei die kardiovaskuläre Erkrankung Kardiomyopathie, Myokardfibrose, Herzrhythmusstörungen oder plötzlichen Herztod umfasst.

10. Verfahren nach einem der Ansprüche 1-6, wobei das Verfahren zu Folgendem dient:
a) Identifizieren mindestens einer Mikroblutung im Herzen des Subjekts;
b) Nachweisen mindestens einer Mikroblutung im Herzen des Subjekts;
c) Prognostizieren mindestens einer Mikroblutung im Herzen des Subjekts; oder
d) Diagnostizieren mindestens einer Mikroblutung im Herzen des Subjekts.

11. Verfahren nach einem der Ansprüche 7-9, wobei das Verfahren zu Folgendem dient:
a) Identifizieren der kardiovaskulären Erkrankung bei dem Subjekts oder Identifizieren, dass das Subjekt die kardiovaskuläre Erkrankung aufweist;
b) Nachweisen der kardiovaskulären Erkrankung bei dem Subjekt;
c) Prognostizieren der kardiovaskulären Erkrankung bei dem Subjekt; oder
d) Bestimmen einer Progression der kardiovaskulären Erkrankung bei dem Subjekt.

12. Verfahren nach Anspruch 1 oder 7, wobei das Subjekt keinen Myokardinfarkt oder keine durch Reperfusionstherapie induzierte Hämorrhagie aufweist oder wobei das Subjekt in den letzten 3 Tagen, 7 Tagen, 1 Monat, 2 Monaten, 3 Monaten, 4 Monaten, 5 Monaten, 6 Monaten, 7 Monaten, 8 Monaten, 9 Monaten, 10 Monaten, 11 Monaten, 12 Monaten, 2 Jahren, 3 Jahren oder 5 Jahren keinen Myokardinfarkt oder keine durch Reperfusionstherapie induzierte Hämorrhagie aufwies.

## Revendications

1. Méthode d'identification, de détection, de pronostic et/ou de diagnostic d'au moins un micro-saignement dans le cœur d'un sujet en ayant besoin, comprenant :
la détection et/ou la mesure de la présence d'au moins un dépôt de fer dans le cœur du sujet en mesurant dans le sang prélevé chez le sujet un taux d'un produit de dégradation de l'hémoglobine ou un taux d'un marqueur de ferroptose,
ladite présence du dépôt de fer dans le cœur du sujet indiquant ledit au moins un micro-saignement dans le cœur du sujet, et
ledit sujet ayant été diagnostiqué avec de l'hypertension, une crise hypertensive, une crise hypertensive aiguë, de l'hypertension prolongée ou une combinaison de celles-ci.

2. Méthode selon la revendication 1, ledit sujet ayant été diagnostiqué avec de l'hypertension.

3. Méthode selon la revendication 1, ladite détection et/ou ladite mesure de la présence dudit au moins un dépôt de fer comprenant la mesure dans le sang prélevé chez le sujet d'un taux d'un produit de dégradation de l'hémoglobine ou d'un taux d'un marqueur de ferroptose, ledit produit de dégradation de l'hémoglobine étant choisi dans le groupe constitué par le fer, la bilirubine, la globine et une combinaison de ceux-ci, et lesdits marqueurs de ferroptose comprenant la glutathion peroxydase 4 (GPX4).

4. Méthode selon la revendication 1, comprenant en outre :
la comparaison du taux du produit de dégradation de l'hémoglobine ou du taux du marqueur de ferroptose du sujet à celui obtenu à partir d'un échantillon de référence.

5. Méthode selon la revendication 4, ladite présence d'au moins un dépôt de fer dans le cœur du sujet par rapport à l'échantillon de référence indiquant, ou un pronostic ou un diagnostic d'au moins un micro-saignement dans le cœur du sujet.

6. Méthode selon la revendication 4 pour déterminer la progression dudit au moins un micro-saignement dans le cœur du sujet, un changement du taux du produit de dégradation de l'hémoglobine ou du taux du marqueur de ferroptose du sujet par rapport à celui obtenu à partir de l'échantillon de référence indiquant la progression d'au moins un micro-saignement dans le cœur du sujet.

7. Méthode d'identification, de détection, de pronostic, de diagnostic et/ou de détermination de la progression d'une maladie cardiovasculaire chez un sujet en ayant besoin, comprenant :
la détection d'au moins un dépôt de fer dans le cœur du sujet en mesurant dans le sang prélevé chez le sujet un taux d'un produit de dégradation de l'hémoglobine ou un taux d'un marqueur de ferroptose, ladite détection du dépôt de fer dans le cœur du sujet indiquant une présence d'au moins un micro-saignement dans le cœur du sujet ; et
la comparaison du taux du produit de dégradation de l'hémoglobine ou du taux du marqueur de ferroptose du sujet à celui obtenu à partir d'un échantillon de référence, un changement du taux du produit de dégradation de l'hémoglobine ou du taux du marqueur de ferroptose du sujet par rapport à celui obtenu à partir de l'échantillon de référence indiquant, ou un pronostic ou un diagnostic d'une maladie cardiovasculaire, ou indiquant la progression de la maladie cardiovasculaire, chez le sujet,
ledit sujet ayant été diagnostiqué avec de l'hypertension, une crise hypertensive, une crise hypertensive aiguë, de l'hypertension prolongée ou une combinaison de celles-ci.

8. Méthode selon la revendication 7, comprenant en outre :
la mise en corrélation dudit au moins un dépôt de fer indiqué par le taux du produit de dégradation de l'hémoglobine ou par le taux du marqueur de ferroptose, du sujet à au moins un micro-saignement dans le cœur du sujet afin d'obtenir une signature de biomarqueur du sujet ; et
ladite étape de comparaison comparant la signature de biomarqueur obtenue du sujet à une signature de biomarqueur provenant d'un échantillon de référence, ce qui permet d'identifier au moins un biomarqueur de la maladie cardiovasculaire, et
un changement de la signature du biomarqueur du sujet par rapport à la signature du biomarqueur de l'échantillon de référence indiquant, ou une détection, un pronostic ou un diagnostic de la maladie cardiovasculaire, ou indiquant la progression de la maladie cardiovasculaire, chez le sujet.

9. Méthode selon la revendication 7, ladite maladie cardiovasculaire comprenant une cardiomyopathie, une fibrose myocardique, une arythmie cardiaque ou une mort cardiaque subite.

10. Méthode selon l'une quelconque des revendications 1 à 6, ledit ladite méthode étant destiné à :
a) identifier au moins un micro-saignement dans le cœur du sujet ;
b) détecter au moins un micro-saignement dans le cœur du sujet ;
c) pronostiquer au moins un micro-saignement dans le cœur du sujet ; ou
d) diagnostiquer au moins un micro-saignement dans le cœur du sujet.

11. Méthode selon l'une quelconque des revendications 7 à 9, ladite méthode étant destiné à :
a) identifier la maladie cardiovasculaire chez le sujet ou identifier un sujet atteint de la maladie cardiovasculaire ;
b) détecter la maladie cardiovasculaire chez le sujet ;
c) pronostiquer la maladie cardiovasculaire chez le sujet ; ou
d) déterminer la progression de la maladie cardiovasculaire chez le sujet.

12. Méthode selon la revendication 1 ou 7, ledit sujet ne présentant pas d'infarctus du myocarde ou d'hémorragie induite par un traitement de reperfusion, ou ledit sujet n'ayant pas présenté d'infarctus du myocarde ou d'hémorragie induite par un traitement de reperfusion au cours des 3 derniers jours, des 7 derniers jours, du dernier mois, des 2 derniers mois, des 3 derniers mois, des 4 derniers mois, des 5 derniers mois, des 6 derniers mois, des 7 derniers mois, des 8 derniers mois, des 9 derniers mois, des 10 derniers mois, des 11 derniers mois, des 12 derniers mois, des 2 dernières années, des 3 dernières années ou des 5 dernières années.
